# FASCICULE DE BREVET EUROPEEN

(11) **EP 3 393 488 B1**
(45) Date de publication et mention de la délivrance du brevet: **25.03.2020**
(21) Numéro de dépôt: 16829274.6
(22) Date de dépôt: 22.12.2016
(51) Int. Cl.: A61K 36/82, A61K 8/49, A61Q 19/00, A61K 8/97, A61K 36/185, A61P 17/04

(54) **COMPOSITION COMPRENANT UN EXTRAIT D'AMBORA ET UN EXTRAIT DE THÉ VERT POUR LE TRAITEMENT DU PSORIASIS, DE LA DERMATITE ATOPIQUE, DE L'URTICAIRE CHRONIQUES, DU PRURIT RESISTANT AUX ANTIHISTAMINIQUES ET DU PRURIT SENILE**
ZUSAMMENSETZUNG MIT EINEM AMBORA-EXTRAKT UND EINEM GRÜNTEE-EXTRAKT ZUR BEHANDLUNG VON PSORIASIS, ATOPISCHER DERMATITIS, CHRONISCHER URTIKARIA, ANTIHISTAMINRESISTENTEM PRURITUS UND SENILEM PRURITUS
COMPOSITION COMPRISING AN AMBORA EXTRACT AND A GREEN TEA EXTRACT FOR THE TREATMENT OF PSORIASIS, ATOPIC DERMATITIS, CHRONIC URTICARIA, ANTIHISTAMINE-RESISTANT PRURITUS AND SENILE PRURITUS

(30) Priorité: 22.12.2015 FR 1563086
(43) Date de publication de la demande: 31.10.2018
(73) Titulaire: Thorel, Jean-Noël, 75014 Paris (FR)
(72) Inventeur: Thorel, Jean-Noël, 75014 Paris (FR)
(74) Mandataire: Cabinet Laurent & Charras
(86) Numéro de dépôt international: PCT/FR2016/053630
(87) Numéro de publication internationale: WO 2017/109423

(56) Documents cités:
- WO-A1-2010/037545
- WO-A2-01/85151
- DATABASE GNPD [Online] MINTEL; juillet 2015 (2015-07), "Mild UV sunscreen for sensitive skin SPF40/PA+++. Dr. CI Labo", XP002759995, Database accession no. 3320437
- DATABASE WPI Week 201379 Thomson Scientific, London, GB; AN 2013-N53411 XP002759996, & KR 2013 0100544 A (BAE Y T) 11 septembre 2013 (2013-09-11) cité dans la demande

## Description

La présente invention se rapporte à une composition cosmétique comprenant une association d'inhibiteurs du facteur de croissance nerveuse (NGF, ou Nerve Growth Factor), particulièrement utile dans le traitement topique du prurit, en particulier le prurit résistant aux antihistaminiques, notamment celui observé dans le cas de psoriasis ou de la dermatite atopique.

Plus précisément, la composition cosmétique comprend des extraits végétaux d'ambora et de thé vert, comme inhibiteurs du facteur de croissance nerveuse (NGF), un facteur impliqué dans les manifestations pathologiques du psoriasis, de la dermatite atopique et dans le prurit et les démangeaisons associés à l'hyperinnervation cutanée.

### ETAT DE LA TECHNIQUE

Le prurit peut être défini comme « une sensation déplaisante qui provoque le besoin de se gratter » (**Misery 2014**). Il survient dans un grand nombre de circonstances, en particulier dans le cas de pathologies cutanées, mais aussi dans le cas d'accumulation de toxines ou de pathologies générales telles les hémopathies ou les maladies endocriniennes.

De manière générale, le prurit produit des démangeaisons persistantes et intraitables qui ont un impact négatif sur la qualité de vie des patients. Le prurit est un symptôme retrouvé notamment dans des pathologies cutanées telles que le psoriasis, la dermatite atopique, ou l'urticaire

Le psoriasis est une maladie inflammatoire chronique caractérisée par une hyperprolifération kératinocytaire et une apoptose réduite, conduisant à un renouvellement épidermique accru. La dermatite atopique (DA), est une dermatose inflammatoire chronique prurigineuse liée à une prédisposition héréditaire du système immunitaire qui s'accompagne d'anomalies de la barrière cutanée. L'atopie cutanée se traduit par une hypersensibilité à des allergènes de l'environnement qui sont normalement tolérés chez des sujets sains.

Dans le cas du psoriasis, le prurit constitue souvent le principal motif de consultation. Sa prise en charge est d'autant plus nécessaire que le grattage entretient l'inflammation comme tout traumatisme (phénomène de Koëbner). De surcroît, le prurit peut entrainer une aggravation de la pathologie, principalement dans le cas de la dermatite atopique. Dans cette pathologie, la fonction barrière de l'épiderme est réduite et le grattage peut compromettre d'autant plus cette première ligne de défense de l'organisme, favorisant la pénétration des allergènes et l'adhésion ou la prolifération des microorganismes pathogènes.

Le prurit est un symptôme typique de l'urticaire, en particulier de l'urticaire chronique, condition caractérisée par la poussée de lésions élémentaires érythémateuses, œdémateuses, bien limitées, en petits éléments ou confluant en larges plaques qui sont mobiles et fugaces. Il n'existe pas de consensus actuel sur le traitement de l'urticaire chronique, qui est généralement prise en charge avec des médicaments antihistaminiques.

Le prurit est souvent aggravé chez les sujets âgés : la diminution de l'épaisseur de la peau, la sécheresse, l'atrophie, la diminution des sécrétions sébacées et sudorales et la diminution du film hydrolipidique de surface jouent un rôle dans l'exacerbation du prurit chez les sujets âgés, de même que la diminution de tous les lipides de la couche superficielle de l'épiderme.

Il est difficile de caractériser outre mesure le prurit, car, comme le phénomène de douleur, il s'agit essentiellement d'un phénomène sensoriel. De plus, les mécanismes moléculaires sous-jacents à son établissement sont complexes et divers. Brièvement, on considère que le prurit est une réponse sensorielle découlant de la stimulation par certains médiateurs des récepteurs sensoriels dits pruricepteurs localisés sur les fibres nerveuses, en particulier les fibres nerveuses de type C situées au niveau de la jonction dermo-épidermique de la peau (**Jacquier 2008**). Les fibres nerveuses de type A δ pourraient elles aussi jouer un rôle dans le prurit.

Classiquement, la prise en charge du prurit est effectuée avec des médicaments antihistaminiques. Un antihistaminique est un médicament utilisé comme antagoniste compétitif des récepteurs de l'histamine, molécule de signalisation du système immunitaire qui est considéré le principal médiateur du prurit. Les antihistaminiques servent donc à réduire ou à éliminer les effets de ce médiateur chimique endogène libéré, par exemple, au cours des réactions allergiques, ou suite à une piqure d'insecte. Cependant, les antihistaminiques sont souvent inefficaces pour traiter des formes spécifiques et récurrentes de prurit. Notamment, dans le cadre du psoriasis, nombreuses études ont montré qu'il n'existe pas de de corrélation entre l'intensité de prurit et le niveau de l'histamine dans le plasma d'individus atteints (**Wisnicka *et al.* 2004**). Dans une autre étude, il a été montré que les antihistaminiques oraux se sont révélés efficaces pour traiter le prurit dans seulement 20% des sujets étudiés atteints de psoriasis (**Szepietowski *et al.* 2002**): cela indique que d'autres voies de signalisation sont impliquées dans l'induction et instauration du prurit, notamment dans le cadre du prurit récurrent.

Des études récentes ont révélé que le facteur de croissance nerveuse (NGF: acronyme de Nerve Growth Factor), une protéine appartenant à la famille des neurotrophines, pourrait jouer un rôle clé en tant que médiateur dans le phénomène du prurit récurrent. Il a été montré que le NGF induit l'expression de la substance P et du CGRP (Calcitonin Gene-Related Peptide) dans les neurones sensoriels. La substance P et le CGRP sont des neuromédiateurs connus pour induire eux aussi le prurit (**Jacquier 2008**). En particulier, la substance P, un neuropeptide appartenant à la classe des tachykinines, est un inducteur puissant du prurit médié par le relargage d'histamine de la part des mastocytes dermiques (**Hägermark *et al.* 1978**).

On sait d'autre part que les neuropeptides tels que la substance P et le CGRP stimulent la prolifération des fibres nerveuses terminales cutanées, qui se traduit par une innervation cutanée plus dense. Ce phénomène a notamment été observé dans les lésions psoriasiques, dans lesquelles le NGF est surexprimé. L'augmentation de la densité de l'innervation cutanée a pour conséquence d'augmenter la sensibilité des sujets aux médiateurs du prurit, en augmentant la présence de récepteurs du prurit. Ceci contribue donc à l'instauration des démangeaisons permanentes et résistantes aux traitements antihistaminiques. Le grattage est la réponse instinctive et naturelle au prurit. Il permet de l'inhiber temporairement, mais le grattage aggrave ensuite la démangeaison, en endommageant le *stratum corneum*, et un cercle vicieux démangeaison-grattage-démangeaison est créé.

Ainsi, bien que les mécanismes moléculaires impliqués soient à l'heure actuelle encore mal compris, le NGF est impliqué dans la sensation de prurit et dans l'instauration d'un prurit récurrent et intraitable à ce jour.

Il y a donc un intérêt à produire des compositions comprenant des inhibiteurs du NGF, qui permettent de limiter voire d'inhiber le prurit. De telles compositions présenteraient un intérêt certain dans le traitement et la prévention du prurit de manière générale, et seraient particulièrement utiles dans le traitement des prurits résistants aux antihistaminiques, notamment des prurits dans lesquels le NGF est surexprimé. Le NGF est retrouvé surexprimé dans la peau dans les processus pathologiques ou physiologiques caractérisés par une hyperprolifération cellulaire, comme c'est le cas notamment lors d'une inflammation, d'une cicatrisation, ou chez les patients atteints de psoriasis ou de dermatite atopique.

Plus précisément, le NGF est surexprimé dans les lésions psoriasiques mais également dans les kératinocytes psoriasiques. De plus, les kératinocytes en culture issus de peau non lésionnelle de patients psoriasiques produisent 10 fois plus de NGF par comparaison avec des kératinocytes de donneurs sains (**Truzzi *et al.* 2011**). Le niveau de NGF est également plus élevé dans les lésions cutanées des personnes atteintes de dermatite atopique. Dans un modèle de co-culture de neurones porcins issus de ganglia rachidiens (DRG) et des cellules de peau humaine, une étude récente a montré que les kératinocytes humains atopiques produisaient des niveaux élevés de NGF associés à une augmentation de CGRP et de la croissance de fibres nerveuses de type C. Il a été suggéré que la surexpression de NGF entraîne une augmentation de la densité de fibres nerveuses épidermiques dans les personnes atteintes de dermatite atopique, avec une exacerbation de la sensation de prurit et démangeaisons accompagnant cette pathologie (**Tominaga *et al.* 2014**).

D'autre part, il a été montré que le NGF est impliqué dans la prolifération des kératinocytes ainsi que dans le phénomène d'inflammation, des symptômes présents notamment dans les lésions psoriasiques. Cette implication serait à la fois directe et indirecte, médiée pour partie par la substance P et le CGRP, dont l'expression est stimulée par le NGF. En effet, chez l'homme, il a été montré que ces neuropeptides jouent un rôle important dans les processus prolifératifs et inflammatoires du psoriasis.

Le NGF se lie notamment au récepteur TrkA, avec une forte affinité. Le récepteur TrkA est un récepteur de type tyrosine kinase. La liaison du NGF induit la phosphorylation du récepteur et l'activation des voies PI3K (Phosphatidylinositol 3-kinase) et PLC (phospholipase C). Via le récepteur TrkA, le NGF produit un effet de stimulation de la prolifération des kératinocytes en culture, de façon dose-dépendante.

D'autre part, le NGF joue un rôle majeur dans les processus inflammatoires neurogènes. Le NGF induit la dégranulation des mastocytes, le recrutement de cellules inflammatoires *via* l'expression d'ICAM-1 dans les cellules endothéliales microvasculaires dermiques et l'activation des Lymphocytes T.

En outre, le NGF possède un effet pro-algique dont l'action semble médiée par le récepteur TRPV1 (acronyme anglais de Transient Receptor Potential Vanilloid-1) (**Nakamura *et al.* 1999)**. En effet, le NGF potentialise l'activité de TRPV1 par phosphorylation et favorise l'insertion du récepteur à la membrane plasmique *via* l'activation de TrkA par un mécanisme impliquant PI3K, PKCδ et Src. En d'autres termes, la surexpression de NGF contribue à l'apparition de symptômes douloureux. Une étude a montré une corrélation positive entre niveaux d'expression de TRPV1 dans l'épiderme, et âge des sujets atteints de prurit, en particulier en réponse aux UV (**Lee** ***et** al.* **2012**). Le prurit sénile est généralement persistant et difficile à traiter : la réduction des niveaux de NGF, couplé avec l'hydratation et la reconstitution de la barrière hydrolipidique peut donc fournir une stratégie de prise en charge efficace du prurit sénile.

Plusieurs études ont montré que les niveaux sériques de NGF sont également élevés chez les sujets atteints d'urticaire (**Bonini *et al.* 1996**), établissant un lien entre urticaire chronique et surexpression de NGF.

Par conséquent, des compositions comprenant des inhibiteurs du NGF pourraient s'avérer très utiles dans le traitement de ces pathologies, certes en diminuant ou inhibant le prurit, mais aussi en limitant l'inflammation et l'hyperkératinisation

Plusieurs approches ont été adoptées pour agir sur la voie du NGF, notamment pour le traitement du prurit, mais aussi des pathologies telles que le psoriasis ou la dermatite atopique, notamment avec pour stratégie d'inhiber l'action du NGF sur son récepteur TrkA.

On peut citer, par exemple, les documents WO 01/85151, WO 2007/022999 et WO 2010/077680 qui divulguent des antagonistes synthétiques du récepteur TrkA, bloquant son activité phosphorylase et/ou kinase.

Par ailleurs, l'utilisation d'extraits végétaux pour le traitement du psoriasis ou de la dermatite atopique est connue.

Par exemple, le document WO 03/057133 décrit l'utilisation d'extraits végétaux tels que des extraits d'*Argemone mexicana* et des extraits de fruits de *Cuminum cyminum,* pour le traitement pharmaceutique du psoriasis.

Les documents CN 1328821, WO 2006/135785 et KR 20130100544 divulguent l'utilisation de polyphénols naturels issus du thé vert ou extrait de thé vert pour la préparation de compositions pour le traitement du psoriasis.

WO 2010/37545 divulgue l'utilisation d'un extrait d'ambora pour le traitement de plusieurs maladies de la peau, en particulier la rosacée.

On trouve aussi des produits cosmétiques, tel que l'écran solaire « Mild UV Sunscreen for sensitive skin SPF40/PA+++ », commercialisé par la société Dr.C : Labo Online, et qui comprend parmis d'autres ingérdients un extrait d'ambora et un extrait de thé vert, dont on ignore s'il est efficace dans le traitement du prurit.

On ignore toutefois les mécanismes mis en jeu par ces compositions, et par suite, leur convenance dans le traitement particulier du prurit associé au psoriasis,à la dermatite atopique, à l'urticaire chronique ou encore dans le traitement du prurit sénile.

Il existe un besoin évident dans la recherche d'alternatives pour le traitement des peaux présentant des prurits résistants aux antihistaminiques, et dans lesquelles la synthèse de NGF est augmentée par rapport à celle d'une peau saine, comme le psoriasis , la dermatite atopique et l'urticaire chronique. Il existe également un besoin pour des traitements qui puissent adresser plusieurs aspects de ces pathologies intraitables, dont notamment le prurit résistant aux antihistaminiques, en particulier le prurit lié à l'hyperinnervation issue de la surexpression de NGF et le prurit sénile.

### EXPOSE DE L'INVENTION

D'une façon surprenante, le Demandeur a mis en évidence que les extraits d'ambora et les extraits de thé vert et leurs combinaisons possèdent plusieurs effets, en particulier un effet inhibiteur sur la surexpression de NGF, un effet inhibiteur de l'hyperinnervation cutanée et un effet sur le relargage de substance P. Ces effets sont particulièrement prononcés lorsque les extraits d'ambora et les extraits de thé vert sont utilisés en combinaison. Ces effets intéressants rendent les extraits et leurs associations particulièrement utiles en tant qu'agent antiprurit, notamment dans le cadre des prurits résistants aux antihistaminiques et du prurit sénile et en tant qu'agent efficace pour traiter le psoriasis, la dermatite atopique et l'urticaire chronique.

Un premier aspect de la présente invention concerne une composition cosmétique comprenant un extrait d'ambora et un extrait de thé vert.

Les extraits d'ambora et les procédés permettant leur obtention sont bien connus de l'homme du métier. L'ambora (*Tambourissa trichophylla*) est arbre ou arbuste persistant endémiques des forets humides du Madagascar. Des extraits d'ambora appropriés dans le cadre de l'invention sont par exemple décrits dans le document WO 2010/037545.

En pratique, l'extrait d'ambora selon l'invention représente de préférence entre 0,00005% et 1% en poids de la composition, avantageusement entre 0,001% et 0,1%, encore plus avantageusement 0,05%.

De préférence, l'extrait d'ambora selon l'invention est un extrait issu des feuilles de la plante. De préférence, l'extrait d'ambora selon l'invention est riche en polyphénols, avantageusement riche en rutine, nicotiflorine, et/ou épicatéchine et des polymères d'acide gallique.

Avantageusement, les polyphénols totaux de l'extrait d'ambora sont constitués majoritairement des tannins d'acide gallique. De préférence, l'extrait d'ambora selon l'invention contient entre 45% et 75% de polyphénols, en poids par rapport au poids de l'extrait.

Un extrait d'ambora adapté à l'invention est un extrait correspondant à la désignation INCI *Tambourissa trichophylla* leaf extract. A titre d'exemple, le produit commercialisé par BAYER HEALTHCARE sous le nom de ROSABORA peut être utilisé dans le cadre de l'invention.

En pratique, l'extrait de thé vert représente de préférence entre 0,00005% et 1% en poids de la composition, avantageusement entre 0,001% et 0,1%, encore plus avantageusement 0,03%.

Les extraits de thé verts sont bien connus de l'homme du métier. Les feuilles du théier (*Camellia sinensis*) notamment celles du thé vert, comprennent plus de 60% de polyphénols en poids de matière sèche. Parmi les polyphénols de la classe des flavanols, sont retrouvés plus particulièrement les catéchines, comme la catéchine, l'épicatéchine, l'épigallocatéchine 3-O-gallate (EGCG). De préférence, l'extrait de thé vert selon l'invention est un extrait de feuilles issues du théier.

De préférence, l'extrait de the vert au sens de l'invention contient plus de 15% de polyphénols de la sous-famille de catéchines en poids par rapport au poids de l'extrait, avantageusement entre 19% et 25%.

De préférence, l'extrait de thé vert selon l'invention comprend de l'épigallocatéchine 3-O-gallate.

Selon un mode de réalisation, l'extrait de thé vert au sens de l'invention contient au moins 13% d'épigallocatéchine 3-O-gallate (EGCG) en poids par rapport au poids de l'extrait.

L'extrait de the vert selon l'invention est, à titre d'exemple, un extrait correspondant à la désignation INCI *Camellia sinensis* leaf extract. A titre d'illustration, le produit commercialisé par INDENA sous le nom de Greenselect® peut être utilisé dans le cadre de l'invention.

La composition de l'invention peut en outre comprendre d'autres extraits végétaux ou composés d'intérêt. De préférence et en fonction de l'application envisagée, la composition comprend, outre l'extrait d'ambora et l'extrait de thé vert, des extraits végétaux ou des composés ayant un effet bénéfique sur le prurit, sur la dermatite atopique ou sur le psoriasis.

Le Demandeur a déterminé que l'association d'extrait de the vert, d'extrait d'ambora et d'acide β-glycyrrhétinique est particulièrement avantageuse dans le cadre d'une composition anti-prurit. En effet, l'acide β-glycyrrhétinique combat rapidement le prurit immédiat causé par la libération d'histamine, tandis que l'extrait de thé vert et l'extrait d'ambora agissent sur le prurit résistant aux antihistaminiques, soulageant durablement la peau.

Ainsi, avantageusement, la composition selon l'invention comprend de l'acide β-glycyrrhétinique.

L'acide β-glycyrrhétinique, ou enoxolone, peut être extrait de la racine de réglisse (*Glycyrrhiza spp*.). En pratique, l'acide β-glycyrrhétinique peut se présenter sous la forme d'un actif pur ou sous forme d'extrait de réglisse titré en acide β-glycyrrhétinique.

De préférence, la composition selon l'invention comprend de l'acide β-glycyrrhétinique purifié de pureté au moins égale à 90%, 95%, avantageusement 98% en poids. A titre d'exemple, le produit tel que commercialisé par MARUZEN, INDENA ou MAFCO sous la dénomination INCI glycyrrhetinic acid peut être utilisé dans le cadre de l'invention.

En pratique, l'acide β-glycyrrhétinique représente de préférence entre 0,001% et 2% en poids de la composition, avantageusement entre 0,01% et 1%, encore plus avantageusement 0,5%.

Le Demandeur a en outre identifié d'autres composés d'intérêt particuliers, lesquels peuvent avantageusement être ajoutés à la composition de l'invention, par exemple pour en améliorer les propriétés. Parmi ces composés, on citera notamment le N-palmitoyl-éthanolamide (PEA), la vitamine B3 et ses dérivés, des lipides aptes à restaurer la barrière cutanée et les composés polyhydroxylés.

Le PEA est bien connu pour exercer des propriétés biologiques en relation avec la douleur chronique et l'inflammation, et a notamment des propriétés anti-inflammatoires, antinociceptives et neuroprotectrices. Le PEA a été impliqué dans l'inhibition du NGF dans un modèle d'hyperalgésie viscérale (**Farguhar-Smith *et al.* 2002**).

De manière avantageuse, la composition de l'invention contient en outre du N-palmitoyl-éthanolamide (PEA). A titre d'exemple, les matières premières cosmétiques ayant pour désignation INCI : palmitamide MEA peuvent être utilisées dans le cadre de l'invention.

En pratique, le N-palmitoyl-éthanolamide représente de préférence entre 0,1% et 1,0% en poids de la composition, avantageusement 0,3%.

La vitamine B3 (aussi connue sous le nom de vitamine PP, niacinamide ou nicotinamide) stimule la synthèse de lipides de la couche cornée, comme les céramides. Elle induit également la synthèse de plusieurs protéines structurales de l'épiderme, comme la filaggrine, l'involucrine et la kératine. La vitamine B3 est un actif hydratant pouvant être utilisé pour lutter contre les états atopiques et les sécheresses cutanées sévères (**Soma 2005**). De nombreuses études scientifiques ont mis en évidence les propriétés anti-inflammatoires de la Vitamine B3 (**Kim *et al.* 2010)**.

De manière avantageuse, la composition contient en outre de la vitamine B3 et/ou un de ses dérivés. A titre d'exemple de dérivés de vitamine B3, on peut citer le tocopheryl nicotinate ou le méthyl nicotinate.

En pratique, la vitamine B3 et/ou un de ses dérivés représentent de préférence entre 0,001% et 2% en poids de la composition, avantageusement entre 0,01% et 0,1%.

Les lipides tels que les constituants naturels du film hydrolipidique, comme le cholestérol, les triglycérides cutanés, les acides gras libres cutanés et les céramides, permettent de restaurer la barrière cutanée. La restauration d'un film hydrolipidique normal réduit l'évaporation de l'eau cutanée, ce qui contribue à combattre les xéroses pre-atopiques.

De manière avantageuse, la composition cosmétique comprend en outre des lipides aptes à restaurer la barrière cutanée, de préférence des constituants naturels du film hydrolipidique, de manière avantageuse choisis parmi le groupe consistant en le cholestérol, le squalane , les triglycérides cutanés, les acides gras libres cutanés et les céramides.

Dans un mode de réalisation avantageux, le lipide apte à restaurer la barrière cutanée est le squalane.

Le squalane au sens de l'invention est avantageusement d'origine végétale. A titre d'exemple, la matière première PHYTOSQUALAN® commercialisée par l'entreprise SOPHIM peut être utilisée dans le cadre de l'invention.

En pratique, les lipides aptes à restaurer la barrière cutanée représentent de préférence entre 0,1% et 5% en poids de la composition, avantageusement entre 1% et 3%.

Les composés polyhydroxylés contribuent à réduire l'adhésion des bactéries pathogènes, comme *S. aureus*, sur la peau et la muqueuse nasale humaine. Le mannitol, en particulier possède une activité antiradicalaire.

De manière avantageuse, la composition cosmétique comprend en outre au moins un composé polyhydroxylé, de préférence choisi dans le groupe consistant en le rhamnose, le xylitol et le mannitol.

Dans un mode de réalisation particulier de l'invention, la composition cosmétique comprend un mélange de rhamnose, xylitol et mannitol. Dans ce mode de réalisation particulier, le rhamnose représente avantageusement entre 0,01% et 1% en poids de la composition, le xylitol représente avantageusement entre 0,05% et 2% en poids de la composition et le mannitol représente avantageusement entre 0,005% et 1% en poids de la composition.

De manière privilégiée, la composition selon l'invention se présente sous une forme adaptée à l'administration par voie topique cutanée: crème, émulsion, H/E ou E/H, solution, suspension, gel, lait, lotion, eau micellaire.

Par conséquent, la présente composition peut contenir tout additif ou excipient adapté à sa formulation et à son application, tel que par exemple agents de mise en suspension, émulsifiants, polymères anioniques, cationiques ou non-ioniques, amphotères, protéines, vitamines, tensioactifs, huiles minérales ou végétales, antioxydants, cires, gommes, résines, agents épaississants, acidifiants ou alcalinisants, stabilisateurs de pH, agents anti-UV, filtres et écrans solaires, conservateurs, parfums, colorants, adjuvants classiques de la cosmétique et de la dermatologie.

Parmi les tensioactifs connus, les tensioactifs non-ioniques sont particulièrement d'intérêt. L'utilisation de tensioactifs non-ioniques permet de réduire l'adhésion et la prolifération du Staphylocoque doré et des levures opportunistes sur la peau ou sur la muqueuse nasale. L'adjonction de ces tensioactifs à la composition est particulièrement avantageuse dans le cadre d'une composition pour traiter la dermatite atopique, laquelle se caractérise par la prolifération d'une flore pathogène.

De manière avantageuse, la composition selon l'invention comprend au moins un tensioactif non-ionique, de préférence choisi dans le groupe consistant en les esters de saccharose, les esters de sorbitan et leurs mélanges. Des esters de saccharose et esters de sorbitan adaptés à cette utilisation sont, par exemple, ceux décrits dans la demande de brevet WO2014/023895A1.

A titre d'exemple, des esters de saccharose appropriés dans le cadre de l'invention sont notamment le sucrose stéarate, le sucrose palmitate, le sucrose laurate.

De préférence, l'ester de saccharose est le sucrose stéarate. Le sucrose stéarate est un mélange d'acide stéarique est d'esters de sucrose. Avantageusement, le sucrose stéarate a une valeur HLB (pour Hydrophobic-lipophilic balance) d'au moins 15, de préférence 16.

De préférence, le sucrose stéarate possède entre 65 et 80% d'acide stéarique en poids par rapport au poids du sucrose stéarate, avantageusement 70%. De préférence, le sucrose stéarate comprend du monoester de sucrose, à hauteur de 70 à 80% en poids par rapport au poids total des esters de sucrose dans le tensioactif, avantageusement 75%.

A titre d'exemple, la matière première SURFHOPE C1816 commercialisée par l'entreprise MITSUBISHI-KAGAKU FOODS CORPORATION peut être utilisée dans le cadre de l'invention.

A titre d'exemple, des esters de sorbitan appropriés dans le cadre de l'invention sont notamment le polysorbate 20, le polysorbate 60, le polysorbate 80.

De préférence, l'ester de sorbitan est le polysorbate 20. A titre d'exemple, la matière première MONTANOX 20 DF commercialisée par la société SEPPIC peut être utilisée dans le cadre de l'invention.

De préférence, l'ester de saccharose et/ou l'ester de sorbitan représente entre 0,01% et 5% en poids de la composition, avantageusement entre 0,05% et 1%.

Il peut être particulièrement avantageux de formuler la composition de l'invention de façon qu'elle soit pulvérisable. Ceci peut être réalisé par exemple par la formulation d'émulsions spécifiques comprenant des combinaisons particulières d'excipients.

Ainsi, de préférence, la composition de l'invention est apte à être pulvérisée.

Ainsi, de préférence, la composition de l'invention est une émulsion et comprend en outre au moins une combinaison d'excipients choisis parmi le groupe consistant en :
- des huiles polaires et/ou des huiles apolaires, et de la gomme gellane, lesdites huiles et ladite gomme étant émulsifiées, de préférence émulsifiées par un sucroester;
- des huiles polaires et/ou des huiles apolaires, et de la gomme gellane, lesdites huiles et ladite gomme étant émulsifiées, de préférence émulsifiées par un acyl glutamate ;
- des huiles polaires et/ou des huiles apolaires, et un polymère acrylate, lesdites huiles et ledit polymère étant émulsifiés, de préférence émulsifiés par un alkyl poly glucoside ;des huiles polaires et/ou des huiles apolaires, de la cellulose microcristalline et un polymère acrylate, lesdites huiles et ladite gomme étant émulsifiées, de préférence émulsifiées par un sucroester.

La composition de l'invention est particulièrement utile dans le traitement d'affections dermatologiques provoquant des prurits.

Ainsi, l'invention vise une composition comprenant un extrait d'ambora et un extrait de thé vert, de préférence pour son utilisation en tant que médicament, de préférence pour son utilisation dans le traitement d'affections dermatologiques provoquant des prurits, de préférence choisie parmi le psoriasis, la dermatite atopique, et l'urticaire chronique et dans le traitement du prurit résistant aux antihistaminiques ou du prurit sénile. Avantageusement, cette utilisation est pour prévenir les récidives de psoriasis, de préférence chez des sujets ayant préalablement suivi un traitement curatif mettant en œuvre des corticoïdes.

La composition selon l'invention est aussi adaptée à toute utilisation classique en cosmétique.

Un autre aspect de l'invention concerne un procédé de traitement cosmétique et/ou de nettoyage des peaux psoriasiques, atopiques ou sujettes au prurit consistant à appliquer sur la peau ou le cuir chevelu une composition comprenant un extrait d'ambora et un extrait de thé vert, de préférence la composition de l'invention.

D'autres buts et aspects avantageux de l'invention ressortiront mieux à la lecture des exemples qui suivent, donnés à titre indicatif et nullement limitatif à l'appui des figures annexées.

### BREVE DESCRIPTION DES FIGURES

La figure 1 illustre le pourcentage d'inhibition du NGF en fonction de différentes concentrations (exprimées en pourcentage de poids par rapport au volume final du milieu de culture) d'extrait d'ambora (A) ou d'extrait de thé vert (B) ou de N-palmitoyl-éthanolamide (C).
La figure 2 illustre les effets de l'association d'un extrait d'ambora et d'un extrait de thé vert sur les prolongements de neurites de neurones sensitifs après traitement par le NGF. La photographie correspond aux images de microscopie prises de kératinocytes cultivées selon les conditions décrites dans l'exemple 2 dans lesquelles les noyaux ont été marqués au DAPI et la bêta tubuline est marquée par immunocytochimie. Le marquage de la bêta-tubuline permet d'observer les neurites, et de mesurer leur longueur.
La figure 3 illustre le Score PASI moyen des rechutes observées dans l'essai clinique chez les sujets traités avec une composition selon l'invention et chez les sujets traités avec le placebo.
La figure 4 illustre l'évolution du score du prurit observé dans l'essai clinique chez les sujets traités avec une composition selon l'invention et chez les sujets traités avec le placebo.
La figure 5 illustre l'impact sur la qualité de vie et le prurit observé dans l'essai clinique chez les sujets traités avec une composition selon l'invention et chez les sujets traités avec le placebo.
La figure 6 illustre l'efficacité d'une composition selon l'invention sur la présence de rougeurs et de squames et sur l'épaisseur de la peau chez les sujets traités.
La figure 7 illustre l'efficacité du placebo sur la présence de rougeurs et de squames et sur l'épaisseur de la peau chez les sujets traités.
La figure 8 illustre les cotations moyennes des critères du test 5D-pruritus à la première visite (première colonne) et après 21 jours de traitement avec le produit de l'invention (deuxième colonne), chez des sujets atteints de psoriasis.
La figure 9 illustre les cotations moyennes des signes cliniques observés à la première visite (première colonne) et après 21 jours de traitement avec le produit de l'invention (deuxième colonne), chez des sujets atteints de psoriasis.
La figure 10 illustre les cotations moyennes des critères du test 5D-pruritus à la première visite (première colonne) et après 21 jours de traitement avec le produit de l'invention (deuxième colonne), chez des sujets atteints d'urticaire chronique.
La figure 11 illustre les cotations moyennes des signes cliniques observés à la première visite (première colonne) et après 21 jours de traitement avec le produit de l'invention (deuxième colonne), chez des sujets atteints d'urticaire chronique.
La figure 12 illustre les cotations moyennes des critères du test 5D-pruritus à la première visite (première colonne) et après 21 jours de traitement avec le produit de l'invention (deuxième colonne), chez des sujets atteints de prurit sénile.
La figure 13 illustre les cotations moyennes des signes cliniques observés à la première visite (première colonne) et après 21 jours de traitement avec le produit de l'invention (deuxième colonne), chez des sujets atteints de prurit sénile.

### EXEMPLES

### Exemple 1/ Effet d'extrait d'ambora, d'extrait de thé vert, et du composé N-palmitoyl-éthanolamide (PEA) sur l'inhibition de l'expression du facteur de croissance nerveuse (NGF).

On détermine de manière quantitative l'effet d'extrait d'ambora, d'extrait de thé vert et du N-palmitoyl-éthanolamide (PEA) sur l'expression de NGF selon la méthode suivante.

### 1.1. Méthode

Cette méthode comprend les étapes suivantes :
- des kératinocytes HaCaT sont ensemencés à une densité de 2,5.10⁴, dans 150 µl de milieu de culture DMEM contenant 10% de sérum de veau fœtal
- les cultures de kératinocytes HaCaT sont incubées avec différentes concentrations des composés à tester, ou en l'absence de composé à tester (contrôle positif de l'expression de NGF) pendant 1h. Un mélange d'inducteurs de l'expression de NGF est ajouté au milieu de culture des cellules. Ce mélange comprend une cytokine pro-inflammatoire (TNFα), et 3 facteurs de croissance (l'EGF, l'IGF-1 et le TGF-α). La concentration de chacun des inducteurs est de 10 ng.ml⁻¹ dans le milieu de culture.
- les cultures de cellules sont incubées en présence du mélange pendant 48h;
- le surnageant des cultures est récupéré;
- la concentration de NGF est mesurée dans les surnageants de culture par ELISA;
- cette concentration est comparée à une concentration témoin, obtenue à partir de la culture n'ayant pas reçu de composé susceptible de diminuer l'expression de NGF (absence de composé à tester).

Pour l'analyse statistique, on prend en compte les résultats d'1 à 6 expériences indépendantes réalisées chacune avec en trois exemplaires identiques de chaque condition testée.

Les analyses quantitatives sont exprimées sous forme de moyenne ± l'écart type. La significativité statistique est déterminée par un test de Student. Une concentration en NGF plus faible dans le surnageant de culture ayant reçu le composé susceptible de diminuer l'expression de NGF comparé à la concentration témoin indique que ledit composé est apte à diminuer l'expression de NGF. Les différences sont considérées comme statistiquement significatives à partir de p<0.05. (NS : p>0.05 ; * : p<0.05 ; ** : p<0.01 ; *** : p<0.001).

### 1.2. Résultats

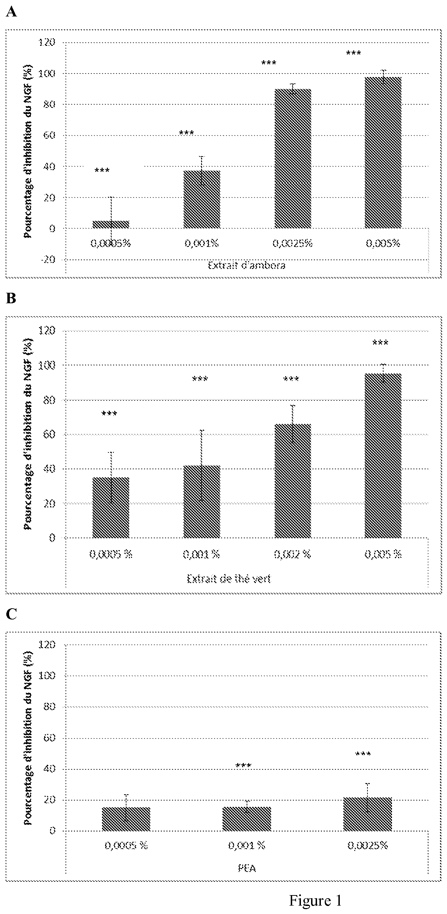

Les résultats des tests ci-dessus sont illustrés aux figures 1A, 1B et 1C.

Les résultats montrent que les extraits d'ambora (figure 1A) et de thé vert (figure 1B) possèdent un effet inhibiteur de l'expression du facteur de croissance nerveuse (NGF), qui croisse avec la dose d'extrait utilisée. A la concentration de 0,005%, une inhibition de plus de 95% est obtenue avec ces deux extraits.

Le PEA possède également un effet inhibiteur sur la synthèse de NGF (figure 1C). Cet effet augmente avec la dose de PEA utilisée.

### Exemple 2/ Effet des mélanges d'extraits végétaux seuls et en association avec le PEA sur l'inhibition de la croissance des neurites (densité et longueurs des fibres) induite par le NGF

Le prurit, notamment celui observé dans le cas de psoriasis ou dermatite atopique, est typiquement associé à une hyperinnervation cutanée corrélée à une surexpression du facteur de croissance nerveuse (NGF) dans les tissus lésés.

Les effets des extraits et composés d'intérêt sur la croissance des neurites ont été évalués sur des cultures de neurones sensitifs après activation par le NGF. La capacité des mélanges testés à inhiber la croissance des neurites témoigne de l'efficacité de ces mélanges à inhiber l'expression du NGF et valide leur intérêt thérapeutique dans le traitement des pathologies susmentionnées.

### 2.1. Méthode

### 2.1.1 Culture des neurones sensitifs

Les neurones sensitifs sont issus de ganglions rachidiens d'embryons de rats de 15 jours. Les ganglions rachidiens isolés sont placés dans du milieu froid Leibovitz contenant 2% de pénicilline 10000 U/mL et Streptomycine 10 mg/mL et 1% de BSA. Les ganglions sont dissociés par trypsination pendant 20 minutes à 37°C. La réaction est stoppée par addition de Dulbecco's modified Eagle's medium (DMEM) contenant de la DNAse I grade II (0,1 mg/ml) et 10% de sérum de veau fœtal. Les cellules sont dissociées mécaniquement par 3 passages dans une pipette 10mL, puis centrifugées à 515 g pendant 10 minutes à 4°C. Le surnageant est éliminé et le culot (comprenant les cellules) est remis en solution dans un milieu DMEM F-12 enrichi (contenant le supplément N2, de la L-glutamine, 2% de PS et 10 ng/mL de Neurotrophine 3 (NT3)). La viabilité des cellules est établie par comptage cellulaire au bleu de trypan. Les cellules sont ensuite ensemencées en plaques de culture de 96 puits traitées avec de la poly L-lysine à raison de 20 000 cellules par puits. Elles sont cultivées dans le milieu DMEM F-12 enrichi susmentionné en étuve à une température de 37°C et sous atmosphère contrôlée (5% de CO2).

### - Pour les tests de cytotoxicité :

Immédiatement après l'ensemencement, les composés à tester sont mis en contact avec les neurones dans un volume final de 190 µL de milieu DMEM F-12 enrichi susmentionné pendant une heure.

On ajoute ensuite 10 µL d'une solution de 100 ng/mL de NGF dans chaque puits de culture, soit une concentration finale de 5 ng/mL dans le milieu de culture. Pour chaque condition, on prépare 3 échantillons (3 puits de culture).

### - Pour l'évaluation de l'efficacité des composés :

Immédiatement après l'ensemencement, les composés à tester sont mis en contact avec les neurones dans un volume final de 190 µL de milieu DMEM F-12 enrichi susmentionné pendant une heure. 10 µL d'une solution de 100 ng/mL de NGF sont ensuite ajoutés dans chaque puits de culture, soit une concentration finale de 5 ng/mL. Pour chaque condition, on prépare 5 échantillons (5 puits de culture). Les cellules sont cultivées pendant 5 jours. Après 2 et 4 jours d'incubation, la moitié du volume de milieu de culture est prélevée et remplacée par du milieu frais, à savoir du DMEM F-12 enrichi contenant les composés à tester et du NGF à 5 ng/mL.

### 2.1.2 Analyse des neurones après 5jours d'incubation en présence des composés

Les différents paramètres suivant sont analysés :
- Numération des corps cellulaires des neurones sensitifs,
- Mesure de la longueur des prolongements des neurites,
- Calcul du ratio longueur des neurites/ nombre de corps cellulaires.

### Préparation des échantillons

Le milieu de culture est éliminé et les cellules sont lavées deux fois au PBS. Les cellules sont fixées par une solution d'éthanol-acide acétique 95-5 pendant 5 minutes à -20°C puis perméabilisées. Les cellules sont incubées dans une solution de PBS contenant 0,1% de saponine et 1% de sérum de veau foetal (SVF) pendant 15 minutes à température ambiante. Cette étape permet de saturer les sites de manière non spécifique. Les cellules sont ensuite incubées 2h avec un anticorps monoclonal de souris anti β-tubuline à la dilution de 1/400è en PBS contenant 1% de SVF et 0.1% de saponine. Cet anticorps marque les neurones sensitifs et leurs prolongements. Cet anticorps est révélé par un anticorps Alexa Fluor 488 chèvre anti-souris pendant 1 heure. Les noyaux cellulaires sont révélés par un marqueur fluorescent (solution de Hoechst).

10 photographies de chaque puit sont réalisées avec le microscope automatique (In Cell 2000 ; GE Healthcare) au grossissement x20.

La numération des corps cellulaires des neurones sensitifs, et la mesure de la longueur des prolongements des neurites sont déterminées grâce au logiciel Analyzer de GE Healthcare. Le nombre de corps cellulaire est exprimé en nombre moyen par puits. La longueur moyenne des prolongements par puits a ensuite été normalisée par le nombre moyen de corps cellulaires dans ce même puits. Les données obtenues sur la longueur des neurites ont été normalisées par rapport à la condition de culture en présence de NGF (sans composés inhibiteurs) qui constitue de manière arbitraire 100% de longueur des neurites. Le calcul du rapport longueur des neurites/ nombre de corps cellulaires est effectué à partir de ces données.

### 2.2. Résultats

Les effets des composés ont été ainsi évalués sur le rapport longueur des neurites/corps cellulaires.

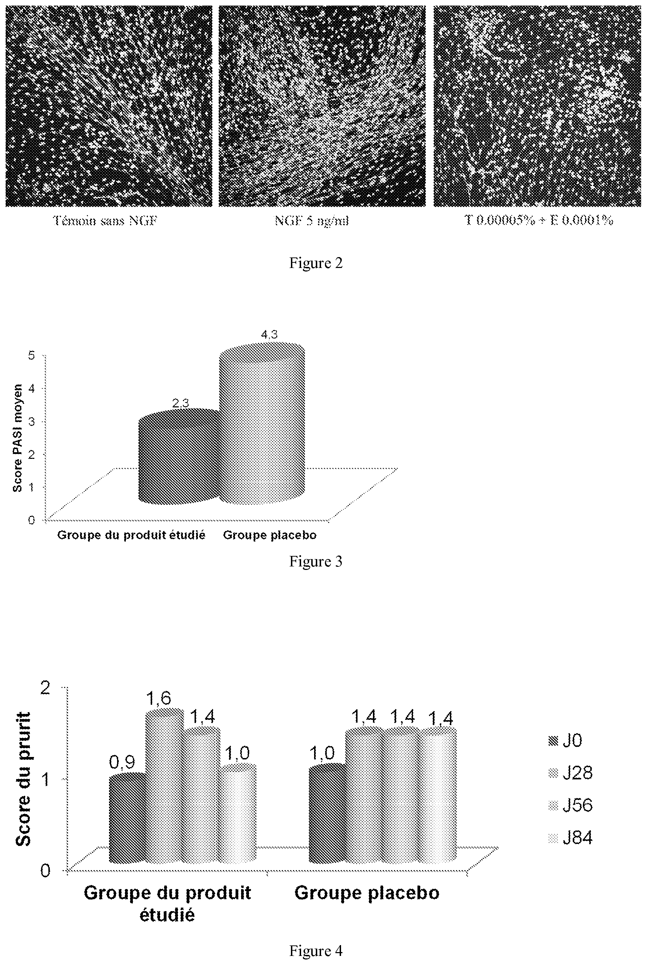

Les résultats des effets des composés sont présentés dans de tableau ci-dessous. Une illustration de ces effets est présentée à la figure 2.

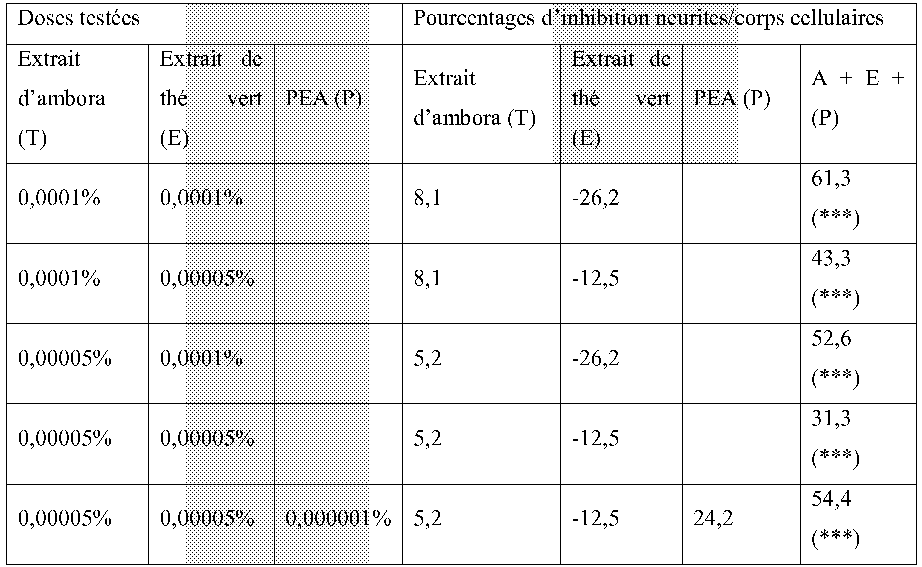

Les résultats obtenus montrent que le NGF à 5 ng/ml augmente significativement la longueur des neurites par rapport aux mêmes cellules cultivées sans NGF. Une augmentation de 40% est observée en présence de NGF, en comparaison avec cellules cultivées sans NGF. Ces résultats valident le modèle utilisé.

Les composés testés ne modifient pas significativement le nombre de corps cellulaires, ce qui confirme qu'ils ne sont pas toxiques.

L'extrait d'ambora inhibe le ratio longueur neurites /corps cellulaires de 5% à 13,5% selon les concentrations, en comparaison avec le contrôle (présence de NGF, absence de composés inhibiteurs). L'extrait de thé vert à la concentration de 0,00001% possède un fort effet inhibiteur avec une réduction de 60,5% des prolongements neuritiques, en comparaison avec le contrôle (présence de NGF, absence de composés inhibiteurs). Les associations d'extrait d'ambora et d'extrait de thé vert inhibent très fortement la longueur des neurites, en comparaison avec le contrôle (présence de NGF, absence de composés inhibiteurs). De manière surprenante des effets synergiques sont observés pour 4 des 5 associations testées. L'ajout du PEA à 0,000001% à cette association de 2 extraits végétaux permet d'augmenter l'efficacité d'inhibition, de manière synergique.

### Exemple 3/ Effet des mélanges d'extraits végétaux sur l'inhibition de l'expression de la substance P dans les neurones sensitifs

Le NGF induit l'expression de substance P, un inducteur puissant du prurit médié par le relargage d'histamine de la part des mastocytes dermiques. La capacité des mélanges testés à inhiber l'expression de substance P témoigne de l'efficacité de ces mélanges à inhiber l'expression du NGF et valide leur intérêt thérapeutique dans le traitement des pathologies susmentionnées.

### 3.1. Méthode

Les cellules sont mises en culture comme décrit précédemment. Les cultures utilisées pour l'immunomarquage décrits au paragraphe 1.1.1 ont été lavées une fois par du PBS contenant 0,1% de saponine et 1% de sérum de veau fœtal (SVF). Les cellules sont ensuite incubées 2h avec un anticorps polyclonal de lapin anti substance P à la dilution de 1/100 en PBS contenant 1% de SVF et 0.1% de saponine. Cet anticorps marque les neurones substance P positifs. Cet anticorps est révélé par un anticorps Alexa Fluor 568 chèvre anti-lapin pendant 1 heure. 10 photographies de chaque puits de culture sont réalisées avec le microscope automatique (In Cell 2000; GE Healthcare) au grossissement x20. La numération des corps cellulaires des neurones sensitifs substance P positifs est réalisée grâce au logiciel Analyzer de GE Healthcare.

### 3.2 Résultats

Les résultats obtenus sont résumés dans le tableau ci-dessous.

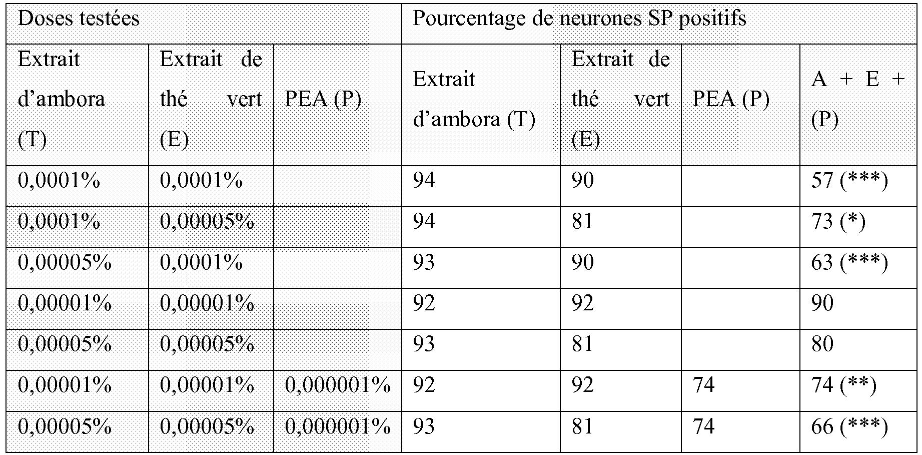

Les résultats obtenus montrent que les cultures incubées avec l'extrait d'ambora, de thé vert et le PEA, ainsi que leurs mélanges, présentent un pourcentage de neurones positifs pour la substance P compris entre 94 et 81%, en comparaison avec le contrôle (présence de NGF, absence de composés inhibiteurs, correspondant à 100% de neurones SP positifs). En conséquence, ces extraits inhibent le nombre de neurones substance P positifs de 6 à 19%, en comparaison avec le contrôle (présence de NGF, absence de composés inhibiteurs).

Les résultats obtenus montrent que l'association d'extraits d'ambora et de thé vert inhibe significativement et de manière synergique le nombre de neurones SP positifs sur 3 des 5 associations, en comparaison avec le contrôle (présence de NGF, absence de composés inhibiteurs).

Les mélanges comprenant un extrait d'ambora, de thé vert et de PEA inhibent significativement le nombre de neurones SP positifs, quelles que soient les concentrations respectives des extraits testés.

### Exemple 4/ crème - émulsion H/E

| ***NOM INCI*** | ***%*** |
|---|---|
| Aqua/Water/Eau | 56,209 |
| Glycerin | 30,00 |
| Coco-Caprylate | 5,00 |
| Mineral oil | 3,00 |
| Tribehenin Peg-20 Esters | 2,50 |
| Sodium Polyacrylate | 1,00 |
| Beta-Sitosterol | 0,50 |
| Fragrance (Parfum) | 0,30 |
| Palmitamide MEA | 0,30 |
| Phospholipids | 0,2625 |
| Disodium EDTA | 0,20 |
| Glycine Soja (Soybean) Oil | 0,1875 |
| Acrylates/C 10-30 Alkyl Acrylate Crosspolymer | 0,15 |
| Xylitol | 0,10 |
| Citric Acid | 0,08 |
| Sodium Metabisulfite | 0,05 |
| *Tambourissa trichophylla* leaf extract | 0,05 |
| Glycolipids | 0,0375 |
| Lecithin | 0,0201 |
| Glycine Soja (Soybean) Sterols | 0,0125 |
| Fructoo ligosaccharides | 0,01 |
| Mannitol | 0,01 |
| *Camellia Sinensis* Leaf Extract | 0,0099 |
| Caprylic/Capric Triglycéride | 0,0095 |
| Rhamnose | 0,001 |
| *Laminaria ochroleuca* Extract | 0,0005 |

Les pourcentages indiqués correspondent au poids du produit indiqué par rapport au poids total de la composition.

### Exemple 5 / gel nettoyant

Les pourcentages indiqués correspondent au poids du produit indiqué par rapport au poids total de la composition.

| ***NOM INCI*** | ***%*** |
|---|---|
| Aqua/water/eau | 86,2225 |
| Sodium laureth sulfate | 3,99 |
| Coco-betaine | 3,90 |
| Methylpropanediol | 2,00 |
| Sodium chloride | 0.91 |
| Pentylene glycol | 0,55 |
| Disodium edta | 0,50 |
| Fragrance (parfum) | 0,50 |
| Sodium citrate | 0,50 |
| Peg-90 glyceryl isostearate | 0,35 |
| Niacinamide | 0,30 |
| Citric acid | 0,14 |
| *Camellia sinensis* leaf extract | 0,05 |
| *Tambourissa trichophylla* leaf extract | 0,05 |
| Laureth-2 | 0,0375 |

### Exemple 6 - spray anti-démangeaisons

Les pourcentages indiqués correspondent au poids du produit indiqué par rapport au poids total de la composition.

| ***NOM INCI*** | ***%*** |
|---|---|
| Aqua/water/eau | 68,1305 |
| Glycerin | 15,00 |
| Dipropylene glycol | 9,00 |
| Isostearyl isostearate | 2,00 |
| Propylheptyl caprylate | 1,999 |
| Squalane | 1,00 |
| Sodium cocoyl glutamate | 0,8825 |
| Glycyrrhetinic acid | 0,50 |
| Pentylene glycol | 0,50 |
| Niacinamide | 0,27055 |
| Disodium EDTA | 0,20 |
| Citric acid | 0,17 |
| Propanediol | 0,125 |
| Xanthan gum | 0,10 |
| *Tambourissa trichophylla* leaf extract | 0,05 |
| Tocopherol | 0,036 |
| Lecithin | 0,0201 |
| *Helianthus annuus* (sunflower) seed oil | 0,015 |
| *Camellia sinensis* leaf extract | 0,0099 |

### Exemple 7/ étude clinique : comparaison de l'efficacité de la composition de l'invention dans le traitement du psoriasis, en particulier sur la sévérité des rechutes du psoriasis, en comparaison avec une formule placebo.

L'effet de la composition de l'invention dans le traitement du psoriasis, en particulier sur la sévérité des rechutes du psoriasis, est testé au cours de l'étude clinique décrite ci-après.

Le produit correspondant à la formulation de l'exemple 4 est testé cliniquement chez des sujets souffrant de psoriasis en plaques léger à modéré, au cours d'une étude monocentrique comparative randomisée en double-aveugle sur deux groupes parallèles, versus placebo.

Le placebo a la formulation suivante :

### Formule du placebo

| ***Nom INCI*** | ***%*** |
|---|---|
| Aqua/Water/Eau | 94,88 |
| Glycerin | 2,00 |
| Hydroxyethyl Acrylate/Sodium Acryloyldimethyl Taurate Copolymer | 1,125 |
| Isohexadecane | 0,765 |
| Titanium Dioxide [Nano] | 0,375 |
| Phenoxyethanol | 0,35 |
| Chlorphenesin | 0,26 |
| Polysorbate 60 | 0,165 |
| Alumina | 0,04 |
| Stearic Acid | 0,035 |

### 7.1 Sélection des sujets de l'étude.

### Critères d'inclusion

- Sujet sain (à l'exception du psoriasis)
- Sujet ayant donné par écrit son consentement libre, éclairé et exprès
- Sujet coopérant, averti de la nécessité et de la durée des contrôles, permettant d'espérer une parfaite adhésion au protocole mis en place par le centre d'essais cliniques
- Sexe : masculin ou féminin
- Âge : supérieur à 18 ans
- Sujet présentant un psoriasis en plaques léger à modéré à J-X (score PASI ≤ 15)
- À J-X : le sujet présente une poussée de psoriasis le jour de la visite et dispose d'une prescription de dermocorticoïdes (pouvant être associés à de l'acide salicylique ou à de l'urée) afin de traiter les plaques de psoriasis
- À J0: le sujet présente une amélioration de son score PASI ≥ 75 % par rapport à J-X
- À J0, chaque score d'intensité d'érythème, d'induration et de desquamation doit être ≤ 1 au niveau de la zone sélectionnée.

### Critères d'exclusion

- Femmes enceintes ou allaitant ou envisageant d'être enceintes lors de l'étude
- Présence d'une pathologie cutanée autre que le psoriasis sur les zones étudiées (selon l'appréciation de l'investigateur)
- Antécédents d'érythrodermie psoriasique, de psoriasis pustuleux ou de psoriasis sévère ayant nécessité une hospitalisation
- Formes autres que le psoriasis en plaques
- Psoriasis sévère nécessitant un traitement systémique
- Sujet résistant aux traitements locaux
- Sujet présentant des signes cliniques d'infection au niveau des zones de test
- Prise d'un traitement topique ou systémique au cours des semaines précédentes susceptible de fausser l'évaluation de la tolérance cutanée du produit étudié (selon l'appréciation de l'investigateur)
- Sujet prenant un traitement de fond contre le psoriasis
- Exposition excessive au soleil, aux rayons UV ou photothérapie au cours du mois précédent et pendant l'étude
- Sujets ayant cessé leur traitement par dermocorticoïdes plus de 3 jours avant l'inclusion (J0)
- Sujets prenant un traitement systémique par immunosuppresseurs, rétinoïdes ou antibiotiques dans les 14 jours précédant le début de l'étude
- Maladie grave pouvant nécessiter une médication systémique régulière (ex. : diabète insulinodépendant, cancer) ou conditions excluant la participation ou pouvant influencer la réaction au test ou son évaluation
- Allergies documentées aux composants des produits étudiés
- Sujet participant à un autre essai clinique au cours de l'étude
- Sujet considéré par l'investigateur comme susceptible de ne pas respecter le protocole.

### 7.2 Déroulement de l'étude

Après un traitement par dermocorticoïdes (de J-X à J0) jusqu'à amélioration du psoriasis, les sujets utilisent soit le la composition de l'invention (formulation de l'exemple 4), soit un placebo. Les produits (composition selon l'invention vs placebo) sont appliqués deux fois par jour pendant trois mois, sur le corps (à l'exception de la tête), sur une peau propre et sèche, dans les conditions normales d'utilisation.

### 7.2.1. Évaluation de la sévérité des rechutes

Les récidives sont documentées. La sévérité de la récidive est évaluée à l'aide du score PASI. Une récidive est définie par une hausse du score PASI (Psoriasis Area Severity Index) de 50 % entre J-X et J-0. Le score PASI est une méthode d'évaluation de la sévérité du psoriasis bien établie, et ne nécessite pas d'être décrit plus précisément. Le score PASI est typiquement compris entre 0 et 72. Dans le cadre de l'étude toutefois, où les sujets n'applique pas de traitement sur la tête, le score PASI est compris entre 0 et 64,8.

A titre d'information, les critères pris en compte dans l'établissement de ce score sont indiqués dans les tableaux ci-après.

**SCORE PASI**

| Score des lésions | Tête (T) | Tronc (Tr) | Membres supérieurs (MS) | Membres inférieurs (MI) |
|---|---|---|---|---|
| Érythème (E) | | | | |
| Induration (I) (épaisseur) | | | | |
| Squames (S) | | | | |
| SOMME: E + I + S | | | | |
| Pourcentage de la zone atteinte | | | | |
| Score surface | | | | |
| SOUS-TOTAL : somme x score surface | | | | |
| Surface corporelle : sous-total x quantités indiquées | X 0,1 | X 0,3 | X 0,2 | X 0,4 |
| TOTAUX | T | Tr | MS | MI |

| | | | | |
|---|---|---|---|---|
| **SCORE PASI (tête exclue) = Tr + MS + MI** | | | | |

*Avec :*

**SCORE DES LÉSIONS**

| Érythème (E) | Aucun symptôme | Légers | Modérés | Sévères | Très sévères |
|---|---|---|---|---|---|
| Induration (I) | | | | | |
| Squames (S) | | | | | |
| SCORE | 0 | 1 | 2 | 3 | 4 |

**SCORE SURFACE :**

| | | | | | | | |
|---|---|---|---|---|---|---|---|
| SURFACE | 0 | 1 % - 9 % | 10 % - 29 % | 30 % - 49 % | 50 % - 69 % | 70 % - 89 % | 90 % - 100 % |
| SCORE | 0 | 1 | 2 | 3 | 4 | 5 | 6 |

### 7.2.2. Évaluation du prurit

Les sujets ont évalué le prurit moyen sur l'ensemble de leur corps à J-X, J0, J28, J56 et J84, à l'aide d'une échelle allant de 0 à 10 (0 : pas de prurit et 10 : prurit très intense).

### 7.2.3. Impact sur la qualité de vie

À J-X, J0, J28, J56 et J84, les sujets ont rempli un questionnaire sur la qualité de vie répertorié et décrit dans différentes publications sur le psoriasis (PDI : Psoriasis Disability Index - Université de Cardiff). Les 15 questions concernent les activités quotidiennes, l'activité professionnelle ou scolaire (si approprié), les relations personnelles, les loisirs et le traitement. Le score maximal de PDI s'élève à 45 et traduit un impact maximal de la maladie sur la qualité de vie.

### 7.2.4. Questionnaire d'évaluation subjective

Les sujets ont rempli un questionnaire d'évaluation subjective à J28, J56 et J84 afin d'évaluer de façon subjective (sur une échelle de 0 à 5) l'efficacité des produits sur leurs rougeurs, leurs squames et l'épaisseur de leurs plaques.

### 7.2.5. Évaluation de la tolérance locale

La tolérance locale du produit a été déterminée à J28, J56 et J84 à l'aide de l'échelle à 5 points suivante : très mauvaise, mauvaise, modérée, bonne, très bonne.

### 7.2.6. Observance

L'investigateur a demandé au sujet à quelle fréquence il a appliqué le produit et pendant combien de jours.

### 7.2.7. Qualités cosmétiques

L'investigateur a questionné les sujets sur les qualités cosmétiques du produit étudié (sur une échelle de 0 à 10) : aspect, texture, couleur, odeur, facilité d'étalement, pénétration dans la peau, vitesse de pénétration.

### 7.3 Résultats

### 7.3.1. Description de la population à l'étude

84 sujets ont été inclus dans l'étude. Sur ce total, 11 personnes ne sont pas allées au terme de l'essai : 9 d'entre elles ont retiré leur consentement, une personne a débuté une grossesse et un autre individu a été sujet à un événement indésirable grave (exacerbation de troubles dépressifs). Selon le protocole, la durée du traitement par corticoïdes devait être comprise entre 4 et 6 semaines. 34 sujets ont reçu un traitement plus court, mais leur participation à l'analyse a été maintenue car l'amélioration de leur score PASI lors de l'inclusion respectait les critères précités. Le traitement s'est étalé sur une période de 6 jours à 6 semaines. De plus, les sujets ont été inclus à J0 à condition que leur score PASI connaisse une amélioration de 75 %. L'un des sujets n'a présenté qu'une hausse de 72 % mais a toutefois été maintenu dans l'analyse, cet écart étant considéré comme mineur. Nous avons également maintenu 11 sujets pour lesquels le délai entre 2 visites a été majoré de ± 7 jours et 14 sujets avec des déviations mineures concernant l'observance. 37 sujets, âgés de 43 ans en moyenne, ont été analysés dans le groupe du produit étudié, et 36 sujets, âgés de 48 ans en moyenne, ont été analysés dans le groupe du placebo. Les sujets présentaient pour la plupart un phototype II (75,7 % dans le groupe du produit étudié et 80,6 % dans le groupe du placebo) et une peau sèche (97,3% dans le groupe du produit étudié et 100% dans le groupe du placebo). Lors de la visite de sélection, le score PASI moyen était de 3,9 dans le groupe du produit étudié et de 4,4 dans le groupe du placebo. Lors de l'inclusion à l'étude, après corticothérapie, ce score a chuté respectivement de 84,6 % (score PASI : 0,6) et de 81,8 % (score PASI : 0,8), dans le groupe du produit étudié et le groupe placebo.

**Description de la population étudiée.**

| | Nombre de sujets | Age | Sexe | PASI à J-X (avant traitement par dermocorticoïdes | PASI à J=0 (après traitement par dermocorticoïdes |
|---|---|---|---|---|---|
| Groupe du produit étudié (composition selon l'invention | 37 | 43±2 ans | F : 59,5% | 3,9±0,3 | 0,6±0,1 |
| | | (Min=21 ans / Max = 69 ans) | H : 40,5% | (de 0,6 à 9,0) | (de 1,0 à 2,0) |
| Groupe placebo | 36 | 48±2 ans | F : 59,3% | 4,4±0,4 | 0,8±0,1 |
| | | (mis 19 / Max = 71 ans) | H : 41,7% | (de 0,8 à 11,2) | (de 1,0 à 2,0) |

Les deux groupes sont comparables du point de vue de leurs caractéristiques générales (âge, sexe, type de peau, phototype) et de la sévérité de leur psoriasis lors de la visite de sélection (J-X) et de leur inclusion à l'étude (J0).

### 7.3.2 Sévérité des rechutes

À J-X (avant le traitement par dermocorticoïdes), la sévérité des rechutes n'était pas statistiquement différente entre les 2 groupes (3,9 pour le produit étudié et 4,4 pour le placebo). De J0 à J84, le PASI moyen évalué lors des rechutes était significativement plus faible dans le groupe du produit étudié par rapport au groupe utilisant le placebo (p = 0,0146 - test de Mann-Whitney), traduisant des rechutes moins sévères avec le produit étudié.

L'utilisation du produit étudié permet de réduire la sévérité des rechutes de 47 % par rapport au placebo (tableau ci-dessous et figure 3).

**Score PASI moyen des rechutes.**

| | Score PASO moyen (±écart-type) |
|---|---|
| Groupe du produit étudié (composition selon l'invention) | 2,3(±0,2) |
| | Min=0,6/Max=4,0 |
| Groupe placebo | 4,3(±0,2) |
| | Min = 0,4/ Max = 19,6 |

### 7.3.3. Efficacité sur le prurit

De 28 à 84 jours après la fin du traitement de base, l'intensité du prurit diminue, quoique de façon non significative. Entre J28 et J84, la réduction de l'intensité du prurit tend à être plus importante avec la composition selon l'invention (- 34,0 %) qu'avec le placebo (- 2,8 %) (figure 4).

### 7.3.4. Questionnaire de qualité de vie (PDI)

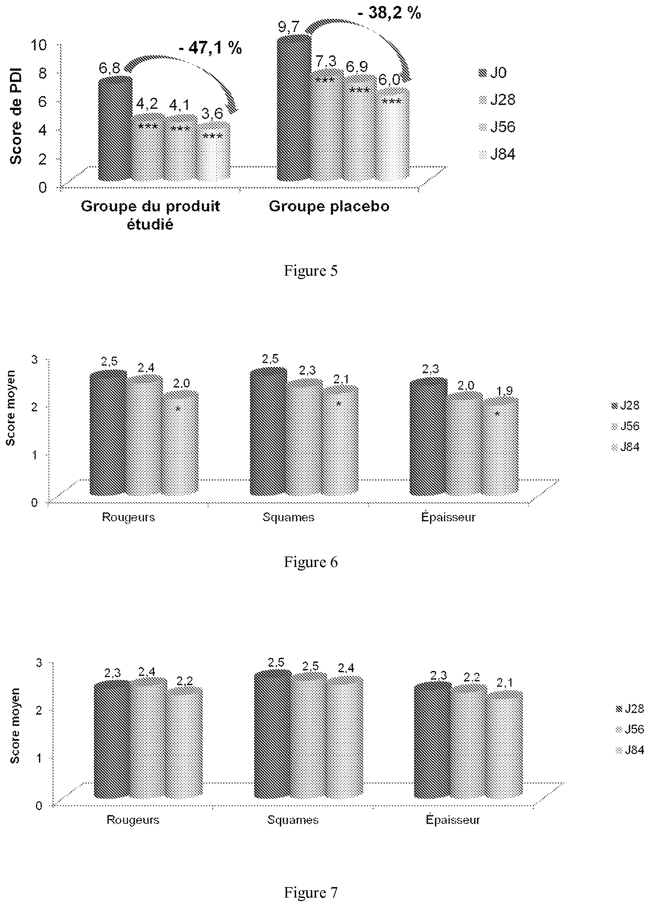

Après 28, 56 ou 84 jours d'utilisation, le score de PDI a enregistré une chute significative pour les deux produits (p < 0,005 - test t non apparié ou test des rangs signés de Wilcoxon) par rapport à la visite d'inclusion (amélioration de la qualité de vie). Cette amélioration est restée assez stable après 56 et 84 jours d'utilisation.
Entre la visite d'inclusion et 84 jours, le score de PDI a davantage baissé avec le produit étudié qu'avec le placebo: diminution de 47,1 % avec la composition selon l'invention et de 38,2 % avec le placebo (figure 5).

### 7.3.5. Efficacité subjective

Les sujets ont constaté une diminution statistiquement significative (p < 0,05 - test des rangs signés de Wilcoxon) de la rougeur, des squames et de l'épaisseur de leurs plaques de psoriasis entre J28 et J84 avec le produit étudié (figure 6)

Aucune variation significative n'a été observée avec le placebo (figure 7).

### 7.3.6. Tolérance Locale

97,3 % des sujets présentent une bonne ou très bonne tolérance au produit étudié. L'un des sujets a ressenti de légers tiraillements sur l'ensemble du corps de J0 à J28. L'investigateur a estimé possible l'imputabilité de cette intolérance au produit. Cette intolérance n'a pas entraîné l'arrêt des applications du produit.
100 % des sujets ont présenté une bonne ou très bonne tolérance au placebo.

### 7.3.7. Qualités cosmétiques du produit

Les qualités cosmétiques du produit étudié, évaluées sur une échelle de 0 à 10 par les sujets, sont jugées bonnes à excellentes.

### 7.4 Conclusions

Cette étude clinique a été réalisée en sur 84 sujets présentant un psoriasis en plaques léger à modéré, pendant 3 mois.

À la suite d'un traitement par dermocorticoïdes ayant conduit à une amélioration du psoriasis, le produit étudié ou le placebo ont été appliqué sur l'ensemble du corps (à l'exception de la tête) deux fois par jour. 37 sujets (âgés de 43 ans en moyenne) ont été analysés dans le groupe du produit étudié contre 36 (âgés de 48 ans en moyenne) dans le groupe placebo.

À la suite d'un traitement par dermocorticoïdes et après 3 mois d'étude, le produit étudié est meilleur que le placebo, après 3 mois d'étude :
- pour réduire significativement la sévérité des rechutes : le score PASI moyen observé lors des rechutes était bien plus faible avec le produit étudié qu'avec le placebo (2,3 contre 4,3), traduisant des rechutes moins sévères avec le produit étudié ;
- pour améliorer les signes cliniques du psoriasis : réduction significative de l'érythème, des squames et de leur épaisseur, stabilisation du prurit ;
- pour améliorer la qualité de vie : réduction de l'impact du psoriasis sur la qualité de vie de 47,1 % avec le produit étudié contre 38,1 % avec le placebo.

Le produit étudié a été bien toléré (97,3 % de bonne ou très bonne tolérance).

Les qualités cosmétiques du produit étudié ont été jugées bonnes à excellentes, permettant ainsi une bonne observance.

En conclusion, le produit étudié permet de maintenir les signes cliniques du psoriasis sous contrôle et peut limiter la sévérité des rechutes.

### Exemple 8/ étude clinique de l'efficacité de la composition de l'invention dans le traitement du psoriasis

L'effet de la composition de l'invention dans le traitement du psoriasis est testé au cours de l'étude clinique décrite ci-après. Le produit correspondant à la formulation de l'exemple 6 est testé cliniquement chez des sujets adultes présentant un psoriasis d'intensité légère à modérée. Il s'agissait pour ces sujets d'utiliser le produit de l'exemple 6 pendant une période de 21 jours. Le relevé des observations par les médecins examinateurs a été réalisé le jour de l'inclusion à V1J0 puis après application régulières à la visite finale V2J21.

### 8.1 Sélection des sujets de l'étude

L'échantillon analysé représente 30 sujets adultes dont 53% de sexe féminin et 47% de sexe masculin, présentant un psoriasis d'intensité légère à modérée. L'âge de ces sujets est compris entre 21 ans et 62 ans et représente une moyenne de 43 ans.

### 8.2 Déroulement de l'étude

Le test s'est déroulé sur une période de 21 jours. Il comprenait 2 visites médicales, l'une prévue avant de commencer le soin (V1J0) et l'autre après 21 jours d'applications régulières du produit (V2J21). Un entretien téléphonique (E1H24) a été réalisé 24h après la visite d'inclusion (V1J0).

Pour chaque sujet inclus dans l'étude, le produit devait être utilisé aussi souvent que nécessaire, en cas de démangeaisons sur la ou les zone(s) concernée(s) pendant 21 jours. En moyenne, le produit a été appliqué 2,5 fois par jour.

Lors des visites, les sujets sont examinés afin de déterminer leur score via le questionnaire 5-D pruritus. Les scores individuels de chaque item (durée, degré et évolution) correspondent à la valeur qui leur a été associée dans le questionnaire (allant de 1 à 5). Les domaines d'invalidités incluent 4 items évaluant l'impact des démangeaisons sur les activités quotidiennes (sommeil, activités sociales, tâches ménagères, travail). Le score d'invalidité est calculé en prenant le score le plus élevé d'un de ces 4 items.

Le nombre de parties du corps affectées par les démangeaisons est pris en compte et la somme définie un score donné :
- Entre 0 et 2 : score de 1
- Entre 3 et 5 : score de 2
- Entre 6 et 10 : score de 3
- Entre 11 et 13 : score de 4
- Entre 14 et 16 : score de 5

Le score final correspond à la somme des scores précédemment obtenus soient :
- La durée
- Le degré
- L'évolution
- L'invalidité
- La localisation

En outre, à chaque visite, un examen clinique est réalisé afin d'analyser l'état cutané des sujets, selon 6 items cliniques et une échelle de cotation à 10 niveaux détaillée dans le tableau ci-dessous.

| Nul | Léger | | | Modéré | | | Important | | |
|---|---|---|---|---|---|---|---|---|---|
| | + | ++ | +++ | + | ++ | +++ | + | ++ | +++ |
| 0 | 1 | 2 | 3 | 4 | 5 | 6 | 7 | 8 | 9 |

Par ailleurs, l'évaluation du contexte personnel a été effectuée à V1J0 et à V2J21 selon 14 items et suivant l'échelle de cotation à 10 niveaux détaillée dans le tableau ci-dessous.

| Nul | Léger | | | Modéré | | | Important | | |
|---|---|---|---|---|---|---|---|---|---|
| | + | ++ | +++ | + | ++ | +++ | + | ++ | +++ |
| 0 | 1 | 2 | 3 | 4 | 5 | 6 | 7 | 8 | 9 |

De plus, le questionnaire SKINDEX a été utilisé pour évaluer la qualité de vie des sujets, selon une échelle de cotation à 5 niveaux allant de 0 : jamais à 4 : tout le temps, détaillée dans le tableau ci-dessous.

| Jamais | Rarement | De temps en | Souvent | Tout le temps |
|---|---|---|---|---|
| 0 | 1 | 2 | 3 | 4 |

Les résultats du SKINDEX sont rapportés en trois scores représentant chacun une dimension donnée de la qualité de vie :
- Émotion
- Symptômes
- Fonctionnement.

Les questions se rapportant au score Emotion sont numérotées : 3, 6, 9, 12, 13, 15, 21, 23, 26, 28. Les questions se rapportant au score Symptômes sont numérotées : 1, 7, 10, 16, 19, 24, 27. Les questions se rapportant au score Fonctionnement sont numérotées : 2, 4, 5, 8, 11, 14, 17, 20, 22, 25, 29, 30.

La question 18 n'est pas incluse dans le calcul des scores.

Le score de chaque dimension est la moyenne des réponses à chaque question de cette dimension. Les scores sont transformés en pourcentage. L'impact en termes de qualité de vie est d'autant plus important que le score est élevé.

En outre, les utilisateurs ont auto-évalué le produit selon plusieurs critères.

Une première autoévaluation du sujet a été recueillie 24 heures après la première utilisation du produit dans 7 items selon une échelle de cotation à 5 niveaux :

| Jamais | Rarement | De temps en | Souvent | Tout le temps |
|---|---|---|---|---|
| 0 | 1 | 2 | 3 | 4 |

L'efficacité perçue est analysée selon les taux de perceptions, regroupés et interprétés comme suit :
- Cotations 3 et 4 (Souvent, Tout le temps) : perceptions « Favorables »,
- Cotation 2 (De temps en temps) : perceptions « Modérées »,
- Cotations 0 et 1 (Jamais, Rarement) : perceptions « Défavorables ».

L'analyse globale de l'efficacité a aussi été évaluée à la visite finale à V2J21, par l'utilisateur selon une échelle de cotation à 5 niveaux (Tout à fait d'accord, D'accord, Pas d'accord, Pas du tout d'accord, Sans Opinion). Les perceptions Favorables correspondent au regroupement des cotations 1 et 2 (Tout à fait d'accord, D'accord) et les évaluations Défavorables au regroupement des cotations 3 et 4 (Pas d'accord, Pas du tout d'accord).

### 8.3 Résultats de l'étude

### 8.3.1 Evaluation du sujet via le questionnaire 5-D pruritus

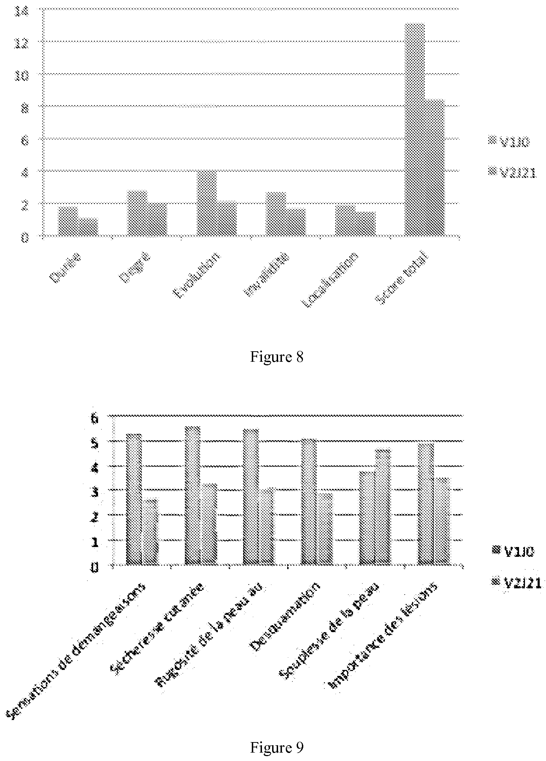

Les résultats de l'évaluation du sujet *via* le questionnaire 5-D pruritus sont présentés dans le tableau ci-dessous et illustrés à la figure 8.

| 5-D pruritus | V1J0 | V2J21 | J21-J0 |
|---|---|---|---|
| Durée | 1,8 | 1,1 | -0,7 (-41%) |
| | | | S* |
| Degré | 2,8 | 2,0 | -0,8 (-27%) |
| | | | S* |
| Evolution | 4,0 | 2,1 | -1,9 (-48%) |
| | | | S* |
| Invalidité | 2,7 | 1,7 | -1,0 (-36%) |
| | | | S* |
| Localisation | 1,9 | 1,5 | -0,4 (-19%) |
| | | | S* |
| Score total | 13,1 | 8,4 | -4,7 (-36%) |
| | | | S* |

La signification statistique, établie sur la comparaison des rangs, est mentionnée à titre indicatif auprès des résultats de l'analyse descriptive. *Signification statistique : NS : non significatif ; S : p<0.05 ; S* : p<0.01; NA : Not Available.

### 8.3.2 Evaluation clinique du sujet

Les résultats de l'évaluation clinique du sujet sont présentés dans le tableau ci-dessous et illustrés à la figure 9.

| Signes cliniques | VIJ0 | V2J21 | J21-J0 |
|---|---|---|---|
| Sensations de démangeaisons | 5,3 | 2,7 | -2,5 (-48%) |
| | | | S* |
| Sécheresse cutanée | 5,6 | 3,3 | -2,3 (-41%) |
| | | | S* |
| Rugosité de la peau au toucher | 5,5 | 3,1 | -2,3 (-43%) |
| | | | S* |
| Desquamation | 5,1 | 2,9 | -2,2 (-43%) |
| | | | S* |
| Souplesse de la peau | 3,8 | 4,7 | 0,9 (25%) |
| | | | S* |
| Importance des lésions cutanées | 4,9 | 3,5 | -1,4 (-29%) |
| | | | S* |

La signification statistique, établie sur la comparaison des rangs, est mentionnée à titre indicatif auprès des résultats de l'analyse descriptive. *Signification statistique : NS: non significatif ; S : p<0.05 ; S* : p<0.01 NA : Not Available.

### 8.3.3 Evaluation du contexte individuel du sujet

Les résultats de l'évaluation du contexte individuel du sujet sont présentés dans le tableau ci-dessous.

| Au cours des 4 dernières semaines, cotation de 0(nul) à 9 (important) | VIJ0 | V2J21 | J21-J0 |
|---|---|---|---|
| Intensité de mon envie de me gratter | 4,8 | 2,4 | -2,4 (-50%) |
| | | | S* |
| Dans quelle mesure cette sensation au grattage m'a limité dans mon travail ou dans mes activités personnelles (domestiques) ? | 3,1 | 1,7 | -1,4 (-46%) |
| | | | S* |
| Mon équilibre a été impacté | 3,0 | 1,6 | -1,4 (-47%) |
| | | | S* |
| Mon problème de peau m'a rendue triste et abattue | 2,2 | 1,3 | -0,9 (-40%) |
| | | | S* |
| Je ne me suis pas sentie plus en confiance | 2,3 | 1,4 | -0,9 (-40%) |
| | | | S* |
| Je me suis sentie plus aimée et appréciée | 1,7 | 1,4 | -0,3 (-18%) |
| | | | NS |
| J'ai eu le goût de m'amuser, de faire du sport, de pratiquer mes loisirs et activités préférés | 3,3 | 4,9 | 1,6 (47%) |
| | | | S* |
| J'ai été plutôt calme et détendue | 3,4 | 4,8 | 1,4 (41%) |
| | | | S* |
| Je me suis sentie débordante d'énergie | 3,4 | 4,9 | 1,4 (42%) |
| | | | S* |
| Je me suis sentie heureux/se | 3,5 | 4,9 | 1,4 (40%) |
| | | | S* |
| Je me suis sentie bien dans ma peau, en paix avec moi-même | 3,0 | 4,7 | 1,7 (56%) |
| | | | S* |
| J'ai eu un bon moral | 3,7 | 5,1 | 1,4 (37%) |
| | | | S* |
| Je me suis sentie en bonne santé, en pleine forme | 3,6 | 5,0 | 1,5 (41%) |
| | | | S* |

La signification statistique, établie sur la comparaison des rangs, est mentionnée à titre indicatif auprès des résultats de l'analyse descriptive. *Signification statistique : NS: non significatif ; S : p<0.05 ; S* : p<0.01 NA : Not Available.

### 8.3.4 Analyse de la qualité de vie des utilisateurs

Les résultats de l'analyse de la qualité de vie des utilisateurs sont présentés dans le tableau ci-dessous.

| SKINDEX SCORE | V1J0 | V2J21 | J21-J0 |
|---|---|---|---|
| Emotions | 16,1 | 10,8 | -5,3 (-33%) |
| | | | S* |
| Symptômes | 12,7 | 7,0 | -5,6 (-44%) |
| | | | S* |
| Fonctionnement | 18,5 | 11,8 | -6,7 (-36%) |
| | | | S* |

La signification statistique, établie sur la comparaison des rangs, est mentionnée à titre indicatif auprès des résultats de l'analyse descriptive. *Signification statistique : NS: non significatif ; S : p<0.05 ; S* : p<0.01 NA : Not Available.

### 8.3.5 Analyse de l'autoévaluation des utilisateurs

Les résultats du taux de perception d'efficacité à 24 heures sont présentés dans le tableau ci-dessous.

| Evaluation à 24h | Fav. | Modéré | Défav |
|---|---|---|---|
| Le produit apaise/soulage la peau dès la 1ère application | 93% | 7% | 0% |
| Le produit diminue l'incitation au grattage dès la 1ère application | 94% | 7% | 0% |
| Le produit diminue la sécheresse cutanée dès la 1ère application | 90% | 10% | 0% |
| Le produit laisse un film protecteur sur la peau dès la 1ère application | 83% | 17% | 0% |
| Le produit hydrate la peau dès la 1ère application | 90% | 10% | 0% |
| Le produit laisse la peau plus douce dès la 1ère application | 90% | 10% | 0% |
| Le produit laisse la peau plus souple dès la 1ère application | 90% | 10% | 0% |

Les résultats du taux de perception d'efficacité à J21 sont présentés dans le tableau ci-dessous.

| Taux des évaluations à J21 | Fav | Défav | SO |
|---|---|---|---|
| Le produit soulage/apaise immédiatement les démangeaisons | 100% | 0% | 0% |
| Le produit diminue durablement les sensations de démangeaisons | 100% | 0% | 0% |
| Le produit diminue l'incitation au grattage | 100% | 0% | 0% |
| Le produit calme les sensations d'irritation | 100% | 0% | 0% |
| Le produit atténue les rougeurs liées au grattage | 100% | 0% | 0% |
| Le produit a une action apaisante sur la peau | 100% | 0% | 0% |
| Le produit atténue l'irritation cutanée | 100% | 0% | 0% |
| Le produit hydrate la peau | 100% | 0% | 0% |
| Le produit nourrit la peau | 97% | 3% | 0% |
| Le produit apporte une sensation de confort | 100% | 0% | 0% |
| Le produit apporte une sensation de confort durable | 100% | 0% | 0% |
| Le produit rend la peau plus douce | 100% | 0% | 0% |
| Le produit rend la peau plus souple | 100% | 0% | 0% |
| Le produit diminue la sécheresse cutanée | 100% | 0% | 0% |
| Le produit laisse un film protecteur sur la peau | 100% | 0% | 0% |

### 8.4 Conclusions

Les résultats obtenus après applications régulières révèlent une amélioration de l'état cutané : soit une diminution des cotations moyennes des sensations de démangeaisons (-48%), de la rugosité de la peau et de la desquamation (-43%), de la sécheresse cutanée (-41%), des lésions cutanées (-29%) et une amélioration de la souplesse de la peau (+25%).

Les comparaisons statistiques des données obtenues à J21 par rapport à J0 confirment ces performances avec pour chacun des critères évalués, une différence significative (p<0.01) en faveur du produit de l'invention.

Les 30 sujets inclus avec un psoriasis d'intensité légère à modérée, hors poussée, présentaient un prurit dont l'évolution a été analysée d'après l'échelle d'évaluation du 5D-pruritus. Après applications répétées du produit, le score global du 5D-pruritus est amélioré (-35%) après 21 jours d'applications répétées avec une différence significative (p<0.01) en faveur du produit de l'invention.

La qualité de vie des sujets a aussi été analysée avant et après 21 jours d'applications répétées selon le questionnaire SKINDEX. Les résultats obtenus révèlent une amélioration de la qualité de vie des sujets dans les 3 dimensions du SKINDEX : symptômes (-44%), fonctionnement (-36%), et émotions (-33%). Les comparaisons statistiques des données obtenues à J21 par rapport à J0 confirment ces performances avec pour chacun des critères évalués, une différence significative (p<0.01) en faveur du produit de l'invention.

Une autoévaluation du produit de l'invention a été réalisée par les utilisateurs après 24 heures afin de juger de l'efficacité immédiate du produit. 94% des utilisateurs estiment que le produit diminue l'incitation au grattage dès la première application. Pour 93% des sujets, le produit apaise, soulage la peau dès la première application. 90% des utilisateurs jugent que dès la première application le produit hydrate la peau, diminue la sécheresse cutanée, laisse la peau plus souple et plus douce dès la première application. Pour 80% des utilisateurs, le produit laisse la peau plus souple dès la première application.

Après 21 jours d'applications répétées, l'efficacité selon les utilisateurs a été évaluée dans 18 items. Les résultats obtenus montrent que 100% des utilisateurs estiment que le produit calme les sensations d'irritation, a une action apaisante sur la peau, diminue l'incitation au grattage et laisse un film protecteur sur la peau. La totalité des utilisateurs jugent que le produit soulage, apaise immédiatement les démangeaisons, atténue l'irritation cutanée, apporte une sensation de confort immédiat, hydrate la peau, diminue durablement les sensations de démangeaisons, atténue les rougeurs liées au grattage, apporte une sensation de confort durable et rend la peau plus douce. Le produit diminue la sécheresse cutanée pour 100% des utilisateurs et il nourrit la peau pour 97% des utilisateurs.

### Exemple 9/ étude clinique de l'efficacité de la composition de l'invention dans le traitement de l'urticaire chronique

L'effet de la composition de l'invention dans le traitement de l'urticaire chronique est testé au cours de l'étude clinique décrite ci-après. Le produit correspondant à la formulation de l'exemple 6 est testé cliniquement chez des sujets adultes présentant une urticaire chronique. Il s'agissait pour ces sujets d'utiliser le produit de l'exemple 6 pendant une période de 21 jours. Le relevé des observations par les médecins examinateurs a été réalisé le jour de l'inclusion à V1J0 puis après application régulières à la visite finale V2J21.

### 9.1 Sélection des sujets de l'étude

L'échantillon analysé représente 30 sujets adultes dont 60% de sexe féminin et 40% de sexe masculin, présentant une urticaire chronique. L'âge de ces sujets est compris entre 18 ans et 82 ans et représente une moyenne de 45 ans.

### 9.2 Déroulement de l'étude

Le test s'est déroulé sur une période de 21 jours. Il comprenait 2 visites médicales, l'une prévue avant de commencer le soin (V1J0) et l'autre après 21 jours d'applications régulières du produit (V2J21). Un entretien téléphonique (E1H24) a été réalisé 24h après la visite d'inclusion (V1J0). Pour chaque sujet inclus dans l'étude, le produit devait être utilisé aussi souvent que nécessaire, en cas de démangeaisons sur la ou les zone(s) concernée(s) pendant 21 jours. En moyenne, le produit a été appliqué 2,3 fois par jour.

Lors des visites, les sujets sont examinés afin de déterminer leur score via le questionnaire 5-D pruritus (critères identiques à ceux détaillés au point 8.2). En outre, à chaque visite, un examen clinique est réalisé afin d'analyser l'état cutané des sujets, selon 6 items cliniques et une échelle de cotation à 10 niveaux identiques à ceux détaillés au point 8.2. Par ailleurs, l'évaluation du contexte personnel a été effectuée à V1J0 et à V2J21 selon 14 items et suivant l'échelle de cotation à 10 niveaux détaillée au point 8.2. De plus, le questionnaire SKINDEX a été utilisé pour évaluer la qualité de vie des sujets, selon une échelle de cotation détaillée au point 8.2 En outre, les utilisateurs ont auto-évalué le produit selon les critères détaillés au point 8.2.

### 9.3 Résultats de l'étude

### 9.3.1 Evaluation du sujet via le questionnaire 5-D pruritus.

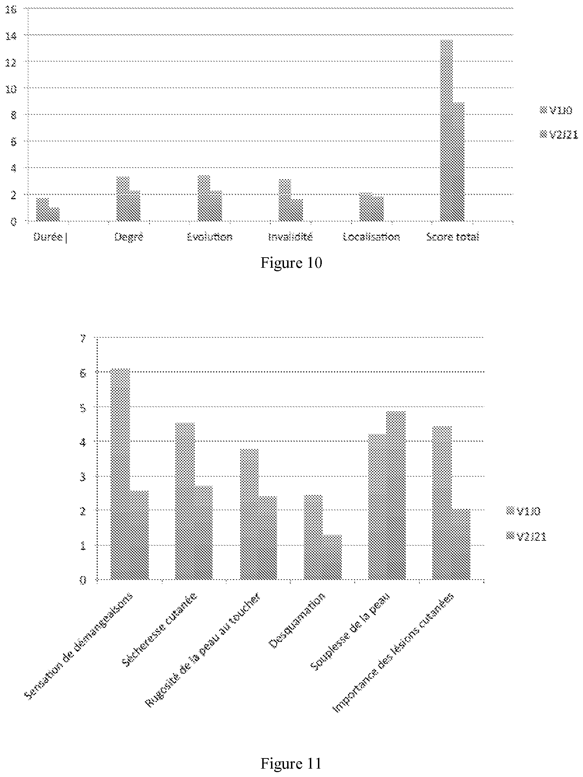

Les résultats de l'évaluation du sujet *via* le questionnaire 5-D pruritus sont présentés dans le tableau ci-dessous et illustrés à la figure 10.

| 5-D pruritus | V1J0 | V2J21 | J21-J0 |
|---|---|---|---|
| Durée | 1,7 | 1,0 | -0,7 (-40%) |
| | | | S* |
| Degré | 3,3 | 2,2 | -1,1 (-32%) |
| | | | S* |
| Evolution | 3,4 | 2,2 | -1,2 (-36%) |
| | | | S* |
| Invalidité | 3,1 | 1,6 | -1,4 (-47%) |
| | | | S* |
| Localisation | 2,1 | 1,8 | -0,3 (-14%) |
| | | | S* |
| Score total | 13,6 | 8,9 | -4,7 (-35%) |
| | | | S* |

La signification statistique, établie sur la comparaison des rangs, est mentionnée à titre indicatif auprès des résultats de l'analyse descriptive. *Signification statistique : NS : non significatif ; S : p<0.05 ; S* : p<0.01; NA : Not Available.

### 9.3.2 Evaluation clinique du sujet

Les résultats de l'évaluation clinique du sujet sont présentés dans le tableau ci-dessous et illustrés à la figure 11.

| Signes cliniques | V1J0 | V2J21 | J21-J0 |
|---|---|---|---|
| Sensations de démangeaisons | 6,1 | 2,6 | -3,5 (-58%) |
| | | | S* |
| Sécheresse cutanée | 4,5 | 2,7 | -1,8 (-40%) |
| | | | S* |
| Rugosité de la peau au toucher | 3,8 | 2,4 | -1,4 (-36%) |
| | | | S* |
| Desquamation | 2,4 | 1,3 | -1,2 (-48%) |
| | | | S* |
| Souplesse de la peau | 4,2 | 4,9 | 0,7 (16%) |
| | | | S* |
| Importance des lésions cutanées | 4,4 | 2,0 | -2,4 (-54%) |
| | | | S* |

La signification statistique, établie sur la comparaison des rangs, est mentionnée à titre indicatif auprès des résultats de l'analyse descriptive. *Signification statistique : NS: non significatif ; S : p<0.05 ; S* : p<0.01 NA : Not Available.

### 9.3.3 Evaluation du contexte individuel du sujet

Les résultats de l'évaluation du contexte individuel du sujet sont présentés dans le tableau ci-dessous.

| Au cours des 4 dernières semaines, cotation de 0(nul) à 9 (important) | VIJ0 | V2J21 | J21-J0 |
|---|---|---|---|
| Intensité de mon envie de me gratter | 5,8 | 2,8 | -3,1 (-53%) |
| | | | S* |
| Dans quelle mesure cette sensation au grattage m'a limitée dans mon travail ou dans mes activités personnelles (domestiques) ? | 3,7 | 1,9 | -1,8 (-49%) |
| | | | S* |
| Mon équilibre a été impacté | 3,3 | 1,2 | -2,1 (-63%) |
| | | | S* |
| Mon problème de peau m'a rendue triste et abattue | 2,5 | 0,9 | -1,6 (-63%) |
| | | | S* |
| Je ne me suis pas sentie plus en confiance | 2,4 | 1,3 | -1,1 (-46%) |
| | | | S* |
| Je me suis sentie plus aimée et appréciée | 1,1 | 0,8 | -0,4 (-32%) |
| | | | NS |
| J'ai eu le goût de m'amuser, de faire du sport, de pratiquer mes loisirs et activités préférés | 3,3 | 5,7 | 2,4 (72%) |
| | | | S* |
| J'ai été plutôt calme et détendue | 3,0 | 5,4 | 2,4 (80%) |
| | | | S* |
| Je me suis sentie débordante d'énergie | 2,9 | 5,3 | 2,3 (80%) |
| | | | S* |
| Je me suis sentie heureux/se | 3,3 | 5,6 | 2,3 (70%) |
| | | | S* |
| Je me suis sentie bien dans ma peau, en paix avec moi-même | 2,4 | 5,4 | 3,0 (123%) |
| | | | S* |
| J'ai eu un bon moral | 3,6 | 5,6 | 2,0 (56%) |
| | | | S* |
| Je me suis sentie en bonne santé, en pleine forme | 3,2 | 5,9 | 2,6 (81%) |
| | | | S* |

La signification statistique, établie sur la comparaison des rangs, est mentionnée à titre indicatif auprès des résultats de l'analyse descriptive. *Signification statistique : NS: non significatif ; S : p<0.05 ; S* : p<0.01 NA : Not Available.

### 9.3.4 Analyse de la qualité de vie des utilisateurs

Les résultats de l'analyse de la qualité de vie des utilisateurs sont présentés dans le tableau ci-dessous.

| SKINDEX SCORE | V1J0 | V2J21 | J21-J0 |
|---|---|---|---|
| Emotions | 13,9 | 7,7 | -6,2 (-45%) |
| | | | S* |
| Symptômes | 12,5 | 6,5 | -6,0 (-48%) |
| | | | S* |
| Fonctionnement | 15,5 | 7,5 | -8,0 (-52%) |
| | | | S* |

La signification statistique, établie sur la comparaison des rangs, est mentionnée à titre indicatif auprès des résultats de l'analyse descriptive. *Signification statistique : NS: non significatif ; S : p<0.05 ; S* : p<0.01 NA : Not Available.

### 9.3.5 Analyse de l'autoévaluation des utilisateurs

Les résultats du taux de perception d'efficacité à 24 heures sont présentés dans le tableau ci-dessous.

| Evaluation à 24h | Fav. | Modéré | Défav |
|---|---|---|---|
| Le produit apaise/soulage la peau dès la 1ère application | 86% | 14% | 0% |
| Le produit diminue l'incitation au grattage dès la 1ère application | 94% | 7% | 0% |
| Le produit diminue la sécheresse cutanée dès la 1ère application | 87% | 10% | 3% |
| Le produit laisse un film protecteur sur la peau dès la 1ère application | 77% | 10% | 13% |
| Le produit hydrate la peau dès la 1ère application | 87% | 10% | 3% |
| Le produit laisse la peau plus douce dès la 1ère application | 86% | 7% | 7% |
| Le produit laisse la peau plus souple dès la 1ère application | 83% | 7% | 10% |

Les résultats du taux de perception d'efficacité à J21 sont présentés dans le tableau ci-dessous.

| Taux des évaluations à J21 | Fav | Défav | SO |
|---|---|---|---|
| Le produit soulage/apaise immédiatement les démangeaisons | 93% | 7% | 0% |
| Le produit diminue durablement les sensations de démangeaisons | 83% | 13% | 3% |
| Le produit diminue l'incitation au grattage | 97% | 3% | 0% |
| Le produit calme les sensations d'irritation | 100% | 0% | 0% |
| Le produit atténue les rougeurs liées au grattage | 80% | 17% | 3% |
| Le produit a une action apaisante sur la peau | 93% | 3% | 3% |
| Le produit atténue l'irritation cutanée | 100% | 0% | 0% |
| Le produit hydrate la peau | 90% | 7% | 3% |
| Le produit nourrit la peau | 63% | 7% | 30% |
| Le produit apporte une sensation de confort immédiat | 93% | 7% | 0% |
| Le produit apporte une sensation de confort durable | 80% | 17% | 3% |
| Le produit rend la peau plus douce | 80% | 13% | 7% |
| Le produit rend la peau plus souple | 67% | 13% | 20% |
| Le produit diminue la sécheresse cutanée | 77% | 13% | 10% |
| Le produit laisse un film protecteur sur la peau 97% | 97% | 3% | 0% |

### 9.4 Conclusions

Les résultats obtenus après applications régulières révèlent une amélioration de l'état cutané : soit une diminution des cotations moyennes des sensations de démangeaisons (-58%), de l'importance des lésions cutanées (-54%), de la desquamation (-48%), de la sécheresse cutanée (-40%), et de la rugosité de la peau au toucher (-36%) et une amélioration de la souplesse de la peau (+16%).

Les comparaisons statistiques des données obtenues à J21 par rapport à J0 confirment ces performances avec pour chacun des critères évalués, une différence significative (p<0.01) en faveur du produit de l'invention.

Les 30 sujets inclus avec une urticaire chronique présentaient un prurit dont l'évolution a été analysée d'après l'échelle d'évaluation du 5D-pruritus. Après applications répétées du produit, le score global du 5D-pruritus est amélioré (-35%) après 21 jours d'applications répétées avec une différence significative (p<0.01) en faveur du produit de l'invention.

La qualité de vie des sujets a aussi été analysée avant et après 21 jours d'applications répétées selon le questionnaire SKINDEX. Les résultats obtenus révèlent une amélioration de la Qualité De Vie des sujets dans les 3 dimensions du SKINDEX : fonctionnement (-52%), symptômes (-48%) et émotions (-45%).

Les comparaisons statistiques des données obtenues à J21 par rapport à J0 confirment ces performances avec pour chacun des critères évalués, une différence significative (p<0.01) en faveur du produit de l'invention.

Une autoévaluation du produit de l'invention a été réalisée par les utilisateurs après 24 heures afin de juger de l'efficacité immédiate du produit. Pour 87% des sujets, le produit apaise, soulage la peau dès la première application et laisse un film protecteur sur la peau dès la première application. 86% des sujets estiment que dès la première application le produit apaise/soulage la peau et hydrate la peau. Le produit laisse la peau plus douce dès la première application pour 83% des sujets. 80% trouvent que le produit laisse la peau plus souple dès la première application et 77% des utilisateurs ont affirmé que le produit diminue la sécheresse cutanée dès la première application.

Après 21 jours d'applications répétées, l'efficacité selon les utilisateurs a été évaluée dans 18 items. Les résultats obtenus montrent que 100% des utilisateurs estiment que le produit calme les sensations d'irritation et a une action apaisante sur la peau. Pour 97% des sujets, le produit diminue l'incitation au grattage et laisse un film protecteur sur la peau. Pour 93% des sujets, le produit soulage, apaise immédiatement les démangeaisons, atténue l'irritation cutanée et apporte une sensation de confort immédiat. 90% des utilisateurs affirment que le produit hydrate la peau. 83% estiment que le produit diminue durablement les sensations de démangeaisons. 80% trouvent que le produit atténue les rougeurs liées au grattage, apporte une sensation de confort durable et rend la peau plus douce. Le produit diminue la sécheresse cutanée pour 77% des utilisateurs. Toutefois, deux items présentent un taux de « Sans opinion » relativement élevé qui exprime une faible pertinence des effets évalués. Ainsi, 30% des utilisateurs n'ont pas d'opinion sur le fait que le produit nourrisse la peau et 20% ne s'expriment pas sur la capacité du produit à rendre la peau plus souple.

### Exemple 10/ étude clinique de l'efficacité de la composition de l'invention dans le traitement du prurit sénile

L'effet de la composition de l'invention dans le traitement du prurit sénile est testé au cours de l'étude clinique décrite ci-après. Le produit correspondant à la formulation de l'exemple 6 est testé cliniquement chez des sujets adultes présentant un prurit sénile. Il s'agissait pour ces sujets d'utiliser le produit de l'exemple 6 pendant une période de 21 jours. Le relevé des observations par les médecins examinateurs a été réalisé le jour de l'inclusion à V1J0 puis après application régulières à la visite finale V2J21.

### 10.1 Sélection des sujets de l'étude

L'échantillon analysé représente 30 sujets adultes dont 60% de sexe féminin et 40% de sexe masculin, présentant un prurit sénile d'intensité légère à modérée. L'âge de ces sujets est compris entre 66 ans et 89 ans et représente une moyenne de 72 ans.

### 10.2 Déroulement de l'étude

Le test s'est déroulé sur une période de 21 jours. Il comprenait 2 visites médicales, l'une prévue avant de commencer le soin (V1J0) et l'autre après 21 jours d'applications régulières du produit (V2J21). Un entretien téléphonique (E1H24) a été réalisé 24h après la visite d'inclusion (V1J0).

Pour chaque sujet inclus dans l'étude, le produit devait être utilisé aussi souvent que nécessaire, en cas de démangeaisons sur la ou les zone(s) concernée(s) pendant 21 jours. En moyenne, le produit a été appliqué 2,9 fois par jour.

Lors des visites, les sujets sont examinés afin de déterminer leur score via le questionnaire 5-D pruritus (critères identiques à ceux détaillés au point 8.2). En outre, à chaque visite, un examen clinique est réalisé afin d'analyser l'état cutané des sujets, selon 6 items cliniques et une échelle de cotation à 10 niveaux identiques à ceux détaillés au point 8.2. Par ailleurs, l'évaluation du contexte personnel a été effectuée à V1J0 et à V2J21 selon 14 items et suivant l'échelle de cotation à 10 niveaux détaillée au point 8.2. De plus, le questionnaire SKINDEX a été utilisé pour évaluer la qualité de vie des sujets, selon une échelle de cotation détaillée au point 8.2 En outre, les utilisateurs ont auto-évalué le produit selon les critères détaillés au point 8.2.

### 9.3 Résultats de l'étude

### 9.3.1 Evaluation du sujet via le questionnaire 5-D pruritus

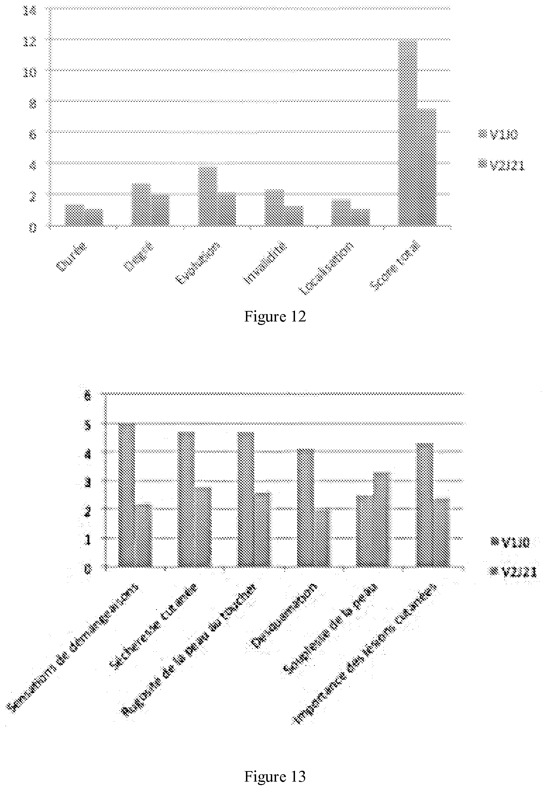

Les résultats de l'évaluation du sujet *via* le questionnaire 5-D pruritus sont présentés dans le tableau ci-dessous et illustrés à la figure 12.

| 5-D pruritus | V1J0 | V2J21 | J21-J0 |
|---|---|---|---|
| Durée | 1,4 | 1,1 | -0,4 (-26%) |
| | | | S* |
| Degré | 2,7 | 2,0 | -0,7 (-26%) |
| | | | S* |
| Evolution | 3,8 | 2,1 | -1,7 (-46%) |
| | | | S* |
| Invalidité | 2,3 | 1,3 | -1,0 (-43%) |
| | | | S* |
| Localisation | 1,7 | 1,1 | -0,6 (-36%) |
| | | | S* |
| Score total | 11,9 | 7,5 | -4,4 (-37%) |
| | | | S* |

La signification statistique, établie sur la comparaison des rangs, est mentionnée à titre indicatif auprès des résultats de l'analyse descriptive. *Signification statistique : NS : non significatif ; S : p<0.05 ; S* : p<0.01; NA : Not Available.

### 10.3.2 Evaluation clinique du sujet

Les résultats de l'évaluation clinique du sujet sont présentés dans le tableau ci-dessous et illustrés à la figure 13.

| Signes cliniques | V1J0 | V2J21 | J21-J0 |
|---|---|---|---|
| Sensations de démangeaisons | 5,0 | 2,2 | -2,8 (-56%) |
| | | | S* |
| Sécheresse cutanée | 4,7 | 2,8 | -1,9 (-41%) |
| | | | S* |
| Rugosité de la peau au toucher | 4,7 | 2,6 | -2,1 (-44%) |
| | | | S* |
| Desquamation | 4,1 | 2,0 | -2,2 (-52%) |
| | | | S* |
| Souplesse de la peau | 2,5 | 3,3 | 0,9 (35%) |
| | | | S* |
| Importance des lésions cutanées | 4,3 | 2,4 | -1,9 (-44%) |
| | | | S* |

La signification statistique, établie sur la comparaison des rangs, est mentionnée à titre indicatif auprès des résultats de l'analyse descriptive. *Signification statistique : NS: non significatif ; S : p<0.05 ; S* : p<0.01 NA : Not Available.

### 10.3.3 Evaluation du contexte individuel du sujet

Les résultats de l'évaluation du contexte individuel du sujet sont présentés dans le tableau ci-dessous.

| Au cours des 4 dernières semaines, cotation de 0(nul) à 9 (important) | VIJ0 | V2J21 | J21-J0 |
|---|---|---|---|
| Intensité de mon envie de me gratter | 5,3 | 2,4 | -3,0 (-56%) |
| | | | S* |
| Dans quelle mesure cette sensation au grattage m'a limitée dans mon travail ou dans mes activités personnelles (domestiques) ? | 1,6 | 0,5 | -1,1 (-67%) |
| | | | S* |
| Mon équilibre a été impacté | 1,8 | 0,6 | -1,2 (-67%) |
| | | | S* |
| Mon problème de peau m'a rendue triste et abattue | 1,7 | 0,4 | -1,3 (-77%) |
| | | | S* |
| Je ne me suis pas sentie plus en confiance | 1,4 | 0,3 | -1,0 (-76%) |
| | | | S* |
| Je me suis sentie plus aimée et appréciée | 0,6 | 0,1 | -0,6 (-89%) |
| | | | S* |
| J'ai eu le goût de m'amuser, de faire du sport, de pratiquer mes loisirs et activités préférés | 2,9 | 4,5 | 1,6 (57%) |
| | | | S* |
| J'ai été plutôt calme et détendue | 3,9 | 5,4 | 1,4 (36%) |
| | | | S* |
| Je me suis sentie débordante d'énergie | 3,8 | 5,4 | 1,6 (42%) |
| | | | S* |
| Je me suis sentie heureux/se | 4,2 | 5,8 | 1,6 (38%) |
| | | | S* |
| Je me suis sentie bien dans ma peau, en paix avec moi-même | 3,3 | 5,7 | 2,4 (72%) |
| | | | S* |
| J'ai eu un bon moral | 4,1 | 5,8 | 1,7 (42%) |
| | | | S* |
| Je me suis sentie en bonne santé, en pleine forme | 4,2 | 5,9 | 1,6 (39%) |
| | | | S* |

La signification statistique, établie sur la comparaison des rangs, est mentionnée à titre indicatif auprès des résultats de l'analyse descriptive. *Signification statistique : NS: non significatif ; S : p<0.05 ; S* : p<0.01 NA : Not Available.

### 10.3.4 Analyse de la qualité de vie des utilisateurs

Les résultats de l'analyse de la qualité de vie des utilisateurs sont présentés dans le tableau ci-dessous.

| SKINDEX SCORE | V1J0 | V2J21 | J21-J0 |
|---|---|---|---|
| Emotions | 12,4 | 4,2 | -8,2 (-66%) |
| | | | S* |
| Symptômes | 14,1 | 6,1 | -8,0 (-57%) |
| | | | S* |
| Fonctionnement | 8,7 | 2,2 | -6,5 (-75%) |
| | | | S* |

La signification statistique, établie sur la comparaison des rangs, est mentionnée à titre indicatif auprès des résultats de l'analyse descriptive. *Signification statistique : NS: non significatif ; S : p<0.05 ; S* : p<0.01 NA : Not Available.

### 9.3.5 Analyse de l'autoévaluation des utilisateurs

Les résultats du taux de perception d'efficacité à 24 heures sont présentés dans le tableau ci-dessous.

| Evaluation à 24h | Fav. | Modéré | Défav |
|---|---|---|---|
| Le produit apaise/soulage la peau dès la 1ère application | 97% | 3% | 0% |
| Le produit diminue l'incitation au grattage dès la 1ère application | 97% | 0% | 3% |
| Le produit diminue la sécheresse cutanée dès la 1ère application | 96% | 3% | 3% |
| Le produit laisse un film protecteur sur la peau dès la 1ère application | 90% | 7% | 3% |
| Le produit hydrate la peau dès la 1ère application | 93% | 7% | 0% |
| Le produit laisse la peau plus douce dès la 1ère application | 93% | 7% | 0% |
| Le produit laisse la peau plus souple dès la 1ère application | 50% | 37% | 13% |

Les résultats du taux de perception d'efficacité à J21 sont présentés dans le tableau ci-dessous.

| Taux des évaluations à J21 | Fav | Défav | SO |
|---|---|---|---|
| Le produit soulage/apaise immédiatement les démangeaisons | 100% | 0% | 0% |
| Le produit diminue durablement les sensations de démangeaisons | 93% | 3% | 3% |
| Le produit diminue l'incitation au grattage | 100% | 0% | 0% |
| Le produit calme les sensations d'irritation | 100% | 0% | 0% |
| Le produit atténue les rougeurs liées au grattage | 97% | 17% | 3% |
| Le produit a une action apaisante sur la peau | 100% | 0% | 0% |
| Le produit atténue l'irritation cutanée | 100% | 0% | 0% |
| Le produit hydrate la peau | 100% | 0% | 0% |
| Le produit nourrit la peau | 93% | 0% | 7% |
| Le produit apporte une sensation de confort immédiat | 100% | 0% | 0% |
| Le produit apporte une sensation de confort durable | 100% | 0% | 0% |
| Le produit rend la peau plus douce | 100% | 0% | 0% |
| Le produit rend la peau plus souple | 77% | 0% | 23% |
| Le produit diminue la sécheresse cutanée | 100% | 0% | 0% |
| Le produit laisse un film protecteur sur la peau | 100% | 0% | 0% |

### 9.4 Conclusions

Les résultats obtenus après applications régulières révèlent une amélioration de l'état cutané : soit une diminution des cotations moyennes des sensations de démangeaisons (-56%), de la desquamation (-52%), de la rugosité de la peau au toucher et de l'importance des lésions cutanées (-44%), de la sécheresse cutanée (-41%) et une amélioration de la souplesse de la peau (+35%).

Les comparaisons statistiques des données obtenues à J21 par rapport à J0 confirment ces performances avec pour chacun des critères évalués, une différence significative (p<0.01) en faveur du produit de l'invention.

Les 30 sujets inclus avec un prurit sénile d'intensité légère à modérée, présentaient un prurit dont l'évolution a été analysée d'après l'échelle d'évaluation du 5D-pruritus. Après applications répétées du produit, le score global du 5D-pruritus est amélioré (-37%) après 21 jours d'applications répétées avec une différence significative (p<0.01) en faveur du produit de l'invention.

La qualité de vie des sujets a aussi été analysée avant et après 21 jours d'applications répétées selon le questionnaire SKINDEX. Les résultats obtenus révèlent une amélioration de la qualité de vie des sujets dans les 3 dimensions du SKINDEX : fonctionnement (-75%), émotions (-66%) et symptômes (-57%).

Les comparaisons statistiques des données obtenues à J21 par rapport à J0 confirment ces performances avec pour chacun des critères évalués, une différence significative (p<0.01) en faveur du produit de l'invention.

Une autoévaluation du produit de l'invention a été réalisée par les utilisateurs après 24 heures afin de juger de l'efficacité immédiate du produit. 97% des utilisateurs estiment que le produit diminue l'incitation au grattage, apaise et soulage la peau dès la première application. Pour 96% des sujets, le produit diminue la sécheresse cutanée dès la première application. 93% des utilisateurs jugent que dès la première application le produit hydrate la peau, et la laisse plus souple. Pour 90% des utilisateurs, le produit laisse un film protecteur sur la peau dès la première application. Seulement 50% des utilisateurs estiment que le produit laisse la peau plus souple dès la première application.

Après 21 jours d'applications répétées, l'efficacité selon les utilisateurs a été évaluée dans 18 items. Les résultats obtenus montrent que 100% des utilisateurs estiment que le produit calme les sensations d'irritation, a une action apaisante sur la peau, diminue l'incitation au grattage et laisse un film protecteur sur la peau. La totalité des utilisateurs jugent que le produit soulage, apaise immédiatement les démangeaisons, atténue l'irritation cutanée, apporte une sensation de confort immédiat, hydrate la peau. Pour 97% des utilisateurs le produit atténue les rougeurs liées au grattage. Le produit nourrit la peau et diminue durablement les sensations de démangeaisons pour 93% des utilisateurs. Toutefois, l'item « le produit rend la peau plus souple» présente un taux de « Sans opinion » relativement élevé (23%) qui exprime une faible pertinence de l'effet évalué.

### RÉFÉRENCES

Bonini et al. (1996) Circulating nerve growth factor levels are increased in humans with allergic disease and asthma Proc. Natl. Acad. Sci USA 93: 10955-10960.
Farquhar-Smith W.P. et al. (2002) Attenuation of nerve growth factor-induced visceral hyperalgesia via cannabinoid CB(1) and CB(2)-like receptors. Pain, 97(1-2):11-21.
Hägermark et al. (1978) Flare and itch induced by substance P in human skin. J Invest Dermatol. Oct;71(4):233-5.
Jacquier C. (2008) Prise en charge du prurit en médecine générale. Thèse pour obtenir le grade de docteur en médecine, université Henri Poincaré Nancy-I, 2008
Kim N.H. et al. (2010) Nicotinamide in dermatology. Expert Review of Dermatology. 5(1): 23-29.
Lee Y.M. et al. (2012) The role of TRPV1 channel in aged human skin. J Dermatol Sci. 65(2):81-85.
Misery L. (2014) Pruritus: considerable progress in pathophysiology, Med. Sci. (Paris): 30(12):1123-1128.
Nakamura A. et al. (1999) Recent advances in neuropharmacology of cutaneous nociceptors". Jpn J Pharmacol. 79(4):427-31.
Raychaudhuri S.P. et al. (2004). Role of NGF and neurogenic inflammation in the pathogenesis of psoriasis Prog Brain Res. 146:433-437.
Soma Y. et al. (2005) Moisturizing effects of topical nicotinamide on atopic dry skin . Int J Dermatol. 44(3):197-202. 200
Szepietowski, C et al. (2002) Itching in patients suffering from psoriasis , Acta Dermat. Croat., 10(4): 221-226.
Tanno O. et al (2000) Niacinamide increases biosynthesis of ceramides as well as other stratum corneum lipids to improve the permeability barrier. Br J Dermatol, 143(3): 524-531.
Truzzi F. et al. (2011) Neurotrophins in healthy and diseased skin. Dermatoendocrinol. 3(1):32-6. doi: 10.4161/derm.3.1.14661.
Tominaga M. et al. (2014) Itch and nerve fibers with special reference to atopic dermatitis: therapeutic implications. J Dermatol. Mar;41(3):205-12. doi: 10.1111/1346-8138.12317.
Wisnicka B. et al. (2004) Histamine, substance P and calcitonin gene-related peptide plasma concentration and pruritus in patients suffering from psoriasis. Dermat. and Psychosom. 5(2): 73-78.

## Revendications

1. Composition cosmétique comprenant un extrait d'ambora et un extrait de thé vert, **caractérisée en ce que** l'extrait d'ambora représente entre 0,00005% et 1%, en poids de la composition.

2. Composition selon la revendication 1, **caractérisée en ce que** l'extrait de thé vert est titré en l'épigallocatéchine 3-O-gallate.

3. Composition selon la revendication 1 ou 2, **caractérisée en ce que** l'extrait de thé vert représente entre 0,00005% et 1% en poids de la composition, avantageusement entre 0,001% et 0,1%.

4. Composition selon l'une des revendications 1 à 3, **caractérisée en ce que** la composition comprend en outre de l'acide β-glycyrrhétinique.

5. Composition selon la revendication 4, **caractérisée en ce que** l'acide β-glycyrrhétinique représente entre 0,001% et 2% en poids de la composition, avantageusement entre 0,01% et 1%.

6. Composition selon l'une des revendications 1 à 3, **caractérisée en ce qu'**elle comprend en outre du N-palmitoyl-éthanolamide.

7. Composition selon la revendication 6, **caractérisée en ce que** le N-palmitoyl-éthanolamide représente entre 0,1% et 1% en poids de la composition.

8. Composition selon l'une des revendications 1 à 7, **caractérisée en ce qu'**elle comprend en outre de la vitamine B3 et/ou un de ses dérivés.

9. Composition selon la revendication 8, **caractérisée en ce que** la vitamine B3 et/ou un de ses dérivés représente entre 0,001% et 2% en poids de la composition.

10. Composition selon l'une des revendications 1 à 9, **caractérisée en ce qu'**elle est apte à être pulvérisée.

11. Composition comprenant un extrait d'ambora et un extrait de thé vert pour son utilisation en tant que médicament dans le traitement d'affections dermatologiques provoquant des prurits, de préférence choisie parmi le psoriasis, la dermatite atopique, et l'urticaire chronique et dans le traitement du prurit résistant aux antihistaminiques ou du prurit sénile.

## Patentansprüche

1. Kosmetische Zusammensetzung mit einem Ambora-Extrakt und einem Grüntee-Extrakt, **dadurch gekennzeichnet, dass** der Ambora-Extrakt zwischen 0,00005 Gewichts-% und 1 Gewichts-% der Zusammensetzung darstellt.

2. Zusammensetzung gemäß Anspruch 1, **dadurch gekennzeichnet, dass** der Grüntee-Extrakt mit Epigallocatechin 3-O-Gallat titriert ist.

3. Zusammensetzung nach Anspruch 1 oder 2, **dadurch gekennzeichnet, dass** der Grüntee-Extrakt zwischen 0,00005 Gewichts-% und 1 Gewichts-% der Zusammensetzung, möglichst zwischen 0,001 Gewichts-% und 0,1 Gewichts-%, darstellt.

4. Verfahren gemäß einem der Ansprüche 1 bis 3, **dadurch gekennzeichnet, dass** die Zusammensetzung außerdem β-Glycyrrhetinsäure enthält.

5. Zusammensetzung gemäß Anspruch 4, **dadurch gekennzeichnet, dass** die β-Glycyrrhetinsäure zwischen 0,001 und 2 Gewichts-% der Zusammensetzung, am besten zwischen 0,01 und 1 Gewichts-%, umfasst.

6. Zusammensetzung gemäß einem der Ansprüche 1 bis 3, **dadurch gekennzeichnet, dass** sie außerdem N-palmitoyl-ethanolamid enthält.

7. Zusammensetzung gemäß Anspruch 6, **dadurch gekennzeichnet, dass** das N-palmitoyl-ethanolamid zwischen 0,1 und 1 Gewichts-% der Zusammensetzung darstellt.

8. Zusammensetzung gemäß einem der Ansprüche 1 bis 7, **dadurch gekennzeichnet, dass** die Zusammensetzung außerdem Vitamin B3 und/oder eines seiner Derivate enthält.

9. Zusammensetzung gemäß Anspruch 8, **dadurch gekennzeichnet, dass** Vitamin B3 und/oder eines seiner Derivate zwischen 0,001 und 2 Gewichts-% der Zusammensetzung darstellt.

10. Zusammensetzung gemäß einem der Ansprüche 1 bis 9, **dadurch gekennzeichnet, dass** sie außerdem pulverisiert werden kann.

11. Zusammensetzung mit einem Ambora-Extrakt und einem Grüntee-Extrakt zum Einsatz als Medikament bei der Behandlung dermatologischer Erkrankungen, die Pruritus verursachen, vorzugsweise wenn es sich um Psoriasis, atopische Dermatitis oder chronische Urtikaria handelt, sowie bei der Behandlung von antihistaminresistentem Pruritus und senilem Pruritus.

## Claims

1. A cosmetic composition comprising an ambora extract and a green tea extract, wherein the ambora extract represents between 0.00005% and 1% by weight of the composition.

2. The composition according to claim 1, wherein the green tea extract is rich in epigallocatechin 3-O-gallate.

3. The composition according to claim 1 or 2, wherein the green tea extract represents between 0.00005% and 1% by weight of the composition, advantageously between 0.001% and 0.1%.

4. The composition according to one of claims 1 to 3, wherein the composition further comprises β-glycyrrhetinic acid.

5. The composition according to claim 4, wherein the β-glycyrrhetinic acid represents between 0.001% and 2% by weight of the composition, advantageously between 0.01% and 1%.

6. The composition according to one of claims 1 to 3, wherein it further comprises N-palmitoyl-ethanolamide.

7. The composition according to claim 6, wherein the N-palmitoyl-ethanolamide represents between 0.1% and 1% by weight of the composition.

8. The composition according to one of claims 1 to 7, wherein it further comprises vitamin B3 and/or a derivative thereof.

9. The composition according to claim 8, wherein vitamin B and/or a derivative thereof represents between 0.001% and 2% by weight of the composition.

10. The composition according to one of claims 1 to 9, wherein it is sprayable.

11. A composition comprising an ambora extract and a green tea extract for use as medicine in the treatment of dermatological disorders causing pruritus, preferably selected from psoriasis, atopic dermatitis, chronic urticaria and in the treatment of antihistamine-resistant pruritus or of senile pruritus.
